(19) Europäisches Patentamt
European Patent Office
Office européen des brevets

(11) **EP 3 555 154 B1**

(12) **EUROPEAN PATENT SPECIFICATION**

(45) Date of publication and mention
of the grant of the patent:
**29.04.2020 Bulletin 2020/18**

(21) Application number: **17826087.3**

(22) Date of filing: **15.12.2017**

(51) Int Cl.:
*C08F 220/06* *(2006.01)*    *C08L 33/02* *(2006.01)*
*C11D 3/37* *(2006.01)*    *C08F 2/38* *(2006.01)*

(86) International application number:
**PCT/US2017/066658**

(87) International publication number:
**WO 2018/118681 (28.06.2018 Gazette 2018/26)**

(54) **MILD OPTICALLY STABLE SURFACTANT COMPOSITIONS**

MILDE, OPTISCH STABILE TENSIDZUSAMMENSETZUNGEN

COMPOSITIONS DE TENSIOACTIFS DOUX OPTIQUEMENT STABLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.12.2016 US 201662435974 P**

(43) Date of publication of application:
**23.10.2019 Bulletin 2019/43**

(73) Proprietor: **Lubrizol Advanced Materials, Inc.**
**Cleveland, OH 44141-3247 (US)**

(72) Inventors:
• **TAMARESELVY, Krishnan**
  **Somerset, NJ 08873 (US)**
• **FILLA, Deborah S.**
  **Cleveland, Ohio 44141-3247 (US)**
• **DASHIELL, David L.**
  **Cleveland, Ohio 44141-3247 (US)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
EP-A1- 2 402 000    WO-A1-2016/205015
US-A1- 2008 113 895

Description

**FIELD OF THE PRESENT TECHNOLOGY**

In one aspect, the present technology relates to mild, optically stable, low pH surfactant compositions comprising low molecular weight, non-crosslinked, linear acrylic copolymers and their use as ocular and/or dermal irritation mitigants in such compositions. Exemplary embodiments of the disclosed technology relate to reduced irritation personal care cleansing compositions, reduced irritation household care cleaning compositions, and reduced irritation industrial and institutional care cleaning compositions that contain a surfactant or surfactants in combination with a non-crosslinked, linear, low molecular weight acrylic copolymer. Such compositions maintain their clarity under low pH and elevated temperature conditions.

**BACKGROUND**

Surfactants are widely used in aqueous based personal care, household care and industrial and institutional care formulations as wetting agents, detergents, and emulsifiers. In personal care cleansing products (e.g., shampoos, body washes, facial cleansers, liquid hand soaps, etc.), household care cleaning products (e.g., hard surface cleaners, laundry detergents, dish soaps, automatic dish washer detergents, shower cleansers, bathroom cleansers, car wash detergents, etc.) and industrial and institutional care cleaners (high strength cleaners, detergents, etc.) the surfactant package is one of the most important components in the detersive formulation. These compositions generally comprise a mixture of one or more surfactants as the active detersive ingredient. The surfactant: 1) improves the wettability of the soiled substrate; 2) loosens soil from the substrate; and 3) emulsifies, solubilizes and/or suspends the loosened soil particles in the aqueous wash medium.

Although in principle any surfactant class (e.g., cationic, anionic, nonionic, amphoteric) is suitable in cleansing or cleaning applications, in practice most personal care cleansers and household cleaning products are formulated with anionic surfactants or with a combination of an anionic surfactant as the primary detersive agent with one or more secondary surfactants selected from the other surfactant classes. Anionic surfactants are often used as detersive agents in cleansers and cleaning products because of their excellent cleaning and foaming properties. From the consumer's perspective, the amount and stability of the foam directly relates to the perceived cleaning efficiency of the composition. Generally speaking, the larger the volume of foam produced and the more stable the foam, the more efficient the perceived cleaning action of the composition. Exemplary anionic surfactants traditionally utilized in these formulations include alkyl sulfates and alkyl benzene sulfonates. While the anionic surfactants and in particular the anionic sulfates and sulfonates are efficient detersive agents and have large foam volume and foam stability properties, they are severe ocular irritants and are capable of causing mild to moderate dermal irritation to some sensitized persons. Accordingly, it has become more and more important to consumers that aqueous cleansing compositions are high foaming as well as mild. These combined properties are especially useful if the cleansing compositions are to be topically applied to human skin and hair. Consequently, efforts have been made to provide cleansing products, such as shampoos, bath and shower gels, and facial cleansers that have these properties. The major problem in providing such products resides in the fact that both properties tend to be mutually incompatible. While high foaming detersive surfactants are generally very harsh, mild surfactants tend to provide insufficient foaming properties.

It is known that the irritation caused by anionic sulfates can be reduced by ethoxylation. However, this reduction in irritation is accompanied by a corresponding reduction in foam volume. For example, sodium lauryl sulfate, a high foaming surfactant, causes significant eye irritation. In contrast, sodium laureth-12 sulfate (the corresponding ethoxylate containing 12 ethoxy groups) is almost completely non-irritating, but is a poor foaming agent (see Schoenberg, "Baby Shampoo," Household & Personal Products Industry 60 (September 1979)). The poor foaming properties of ethoxylated alkyl sulfates are reported in many other publications. For example, U.S. Patent No. 4,132,678 discloses that the foaming properties of alkyl ($C_{10}$ to $C_{18}$) sulfates are drastically reduced if more than 5 ethoxy groups are added to the molecule. Additional attempts to attenuate the adverse irritant effects of anionic surfactants have been made by replacing some of the foam generating anionic surfactant with very mild secondary surfactants. The anionic surfactant is utilized in conjunction with a nonionic and/or an amphoteric surfactant as disclosed in U.S. Patent No. 4,726,915. However, reducing the amount of anionic surfactant in a cleansing or cleaning composition adversely affects the detersive and foaming properties of the composition.

Another approach for attenuating the adverse irritant effects of anionic detersive surfactants while maintaining high cleansing and foaming properties as well as ideal product viscosities in personal care cleansing compositions is disclosed in U.S. Patent Nos. 7,803,403; 8,025,902; and 8,293,845 and EP2402000. These patents disclose linear (non-crosslinked) acrylic copolymer materials that are capable of binding surfactants and are utilized in surfactant containing personal care compositions to mitigate irritation to the skin and eyes. The disclosed linear acrylic copolymers are of a low molecular weight ($\leq$100,000 Daltons ($M_n$)) and are comprised of repeating unit residues of a first monomer selected from one or more $\alpha,\beta$-ethylenically unsaturated monomers containing at least one carboxylic acid group and a second hydrophobically modified monomer selected from one or more $\alpha,\beta$-ethylenically unsaturated non-acid monomers containing a $C_1$ to $C_9$ alkyl group, including $C_1$ to $C_9$ alkyl esters of (meth)acrylic acid.

The amount of the first monomeric component to the second monomeric component utilized to prepare the disclosed copolymer ranges from 20:80 wt.% to 50:50 wt.%, based on the weight of the total monomers. The exemplified linear acrylic copolymer identified as EX-968 in Example 1 of U.S. '403 and U.S. '903 is prepared from methacrylic acid and ethyl acrylate in a ratio of 25:75 methacrylic acid:ethyl acrylate, and as set forth in Tables 5 and 6 polymer EX-968 maintains excellent clarity when added to clear surfactant bases. Example 13 in Table 2 of U.S. '845 discloses a surfactant composition with good clarity containing a linear acrylic copolymer prepared from methacrylic acid and ethyl acrylate having a weight ratio of methacrylic acid:ethyl acrylate of 73:27 wt.%. While hydrophobically modified linear copolymers having up to 50 wt.% of repeating unit residues of an $\alpha,\beta$-ethylenically unsaturated monomer containing at least one carboxylic acid group are known to mitigate irritation caused by harsh surfactant containing compositions while maintaining the clarity of clear systems, a drawback of these polymers is that the optical clarity attributes deteriorates in low pH environments and when exposed to prolonged elevated temperatures. These instability problems directly impacts the long term shelf life aesthetics of products containing these irritation mitigant polymers.

While these compositions are typically stable at ambient temperatures, the optical clarity of these formulations decreases when exposed to prolonged elevated temperatures of about 45°C and above. Consequently, care must be taken when transporting and storing these products in warm and tropical regions of the world. Additionally, while these polymers offer good clarity properties in surfactant containing formulations at pH values $\geq 5.0$ they become hazy at acidic pH ranges, resulting in poor clarity.

Microbial contamination from bacteria, yeast, and/or fungus in cosmetics, toiletries and personal care products is very common and has been of great concern to the industry for many years. Present day surfactant containing products are typically formulated with a preservative to protect the product from microbial contamination, decay, discoloration, or spoilage and to ensure that the product is safe for topical application to the skin, scalp, and hair in humans and animals. Three classes of preservative compounds that are commonly used in surfactant containing products are: 1) formaldehyde donors such as diazolinyl urea, imidazolinyl urea, and DMDM Hydantoin; 2) halogenated compounds including 2,4-dichlorobenzyl-alcohol, Chloroxylenol (4-chloro-3,5-dimethyl-phenol), Bronopol (2-bromo-2-nitropropane-1,3-diol), and iodopropynyl butyl carbamate; and 3) the paraben compounds including methyl-paraben, ethyl-paraben, propyl-paraben, butyl-paraben, isopropyl-paraben, and benzyl-paraben.

While these preservatives have been successfully utilized in personal care products for many years, there are recent concerns by the scientific community and the public that some of these compounds may constitute health hazards. Accordingly, there is an interest in replacing the above-mentioned compounds in surfactant containing products that are topically applied to or come into contact with human skin, scalp or hair while maintaining good antimicrobial efficacy, mildness, and do not raise safety concerns.

Organic acids (e.g., sorbic, citric and benzoic), such as those used as preservatives in the food industry, have been increasingly looked at as the ideal replacement for foregoing preservative systems in surfactant containing formulations. The antimicrobial activity of the organic acids is connected to the associated or protonated species of the acid molecule. As the pH of an organic acid containing formulation increases, dissociation of the proton occurs forming acid salts. The dissociated form of the organic acids (acid salts) have no antimicrobial activity when used alone, effectively limiting the use of organic based acids to pH values below 6 (Weber, K. 2005. New alternatives to paraben-based preservative blends. Cosmetics & Toiletries 120(1): 57-62).

The literature has also suggested that formulating products in the natural pH range (between about 3-5) 1) reduces the amount of preservative required in a product by enhancing preservative efficacy; 2) stabilizes and increases the effectiveness of many cosmetic active ingredients; 3) is beneficial to the repair and maintenance of skin barrier tissue; and 4) supports the natural skin flora to the exclusion of over-colonization by deleterious microorganisms (Wiechers, J.W. 2008. Formulating at pH 4-5: How lower pH benefits the skin and formulations; Cosmetics & Toiletries 123(12): 61-70).

As the industry desires new mild surfactant based products that are formulated in the acidic pH range, there is a developing need for an irritation mitigant polymer that, when used in combination with a surfactant, provides a high clarity formulation in low pH conditions as well as optical stability under prolonged exposure to high temperatures. Accordingly, there is a need for an irritation mitigant that does not significantly change the ideal viscosity and optical clarity profile of a surfactant containing composition.

## SUMMARY OF THE DISCLOSED TECHNOLOGY

The present technology provides mild, optically stable cleansing compositions and methods of reducing the irritation associated with a variety of personal care, home care and animal care compositions. In particular, according to certain aspects of the present technology, applicants have discovered advantageously that non-crosslinked, linear acrylic copolymers capable of binding surfactant thereto can be used to produce mild, optically stable surfactant containing compositions exhibiting surprisingly low dermal and/or ocular irritation. The mild surfactant compositions of the present technology exhibit high clarity under low pH conditions and/or prolonged exposure to temperatures as high as 45°C, as compared to compositions comprising comparable polymeric materials.

In one aspect, the present technology provides mild, optically stable surfactant compositions comprising a non-crosslinked linear emulsion acrylic copolymer having a number average molecular wt. ranging from about 10,000 to 100,000 Daltons, said polymer comprises, consists essentially of, or consists of from 51 to 75 wt.% of polymerized residues of at least one $\alpha,\beta$-ethylenically unsaturated $C_3$-$C_6$ carboxylic acid monomer, from 25 to 49 wt.% of polymerized residues of a $C_1$-$C_4$ alkyl (meth)acrylate monomer, and 0, 0.05, 0.1, 0.3 or 0.5 to 1 wt.% of a residue of a chain transfer agent (based on 100 wt. parts of monomer).

In one aspect, the present technology provides mild, optically stable surfactant compositions comprising a non-crosslinked, linear emulsion acrylic copolymer a number average molecular wt. ranging from about 10,000 to 100,000 Daltons, said polymer comprises, consists essentially of, or consists of from 51 to 75 wt.% of polymerized residues of a monomer selected from (meth)acrylic acid, and from 25 to 49 wt.% of polymerized residues of a monomer selected from ethyl (meth)acrylate, butyl (meth)acrylate, and mixtures thereof, and 0, 0.05, 0.1, 0.3 or 0.5 to 1 wt.% of a residue of a chain transfer agent (based on 100 wt. parts of monomer).

In one aspect, the present technology provides mild, optically stable surfactant compositions comprising a non-crosslinked linear emulsion acrylic copolymer having a number average molecular wt. ranging from about 10,000 to 100,000 Daltons, said polymer comprises, consists essentially of, or consists of from 51 to 75 wt.% of polymerized residues of methacrylic acid, and from 25 to 49 wt.% of polymerized residues of ethyl acrylate, and 0, 0.05, 0.1, 0.3 or 0.5 to 1 wt.% of a residue of a chain transfer agent (based on 100 wt. parts of monomer).

## DESCRIPTION OF EXEMPLARY EMBODIMENTS

As used herein the term "low molecular weight" polymer refers to a polymer having a number average molecular weight ($M_n$) as measured by gel permeation chromatography (GPC) calibrated with a poly(methyl methacrylate) (PMMA) standard of about 100,000 Daltons or less. In one aspect, low molecular weight polymers are those having molecular weight ranges of from about 10,000 to about 100,000 Daltons ($M_n$), from about 25,000 to about 80,000 Daltons ($M_n$) from about 40,000 and 75,000 Daltons ($M_n$) and from about 50,000 to about 70,000 Daltons ($M_n$).

As used herein the term "low pH" refers to a pH value of 5.5 or less. In one aspect low pH refers to a pH value ranging from about 2 to about 5.5, from about 2.5 to about 5, from about 3 to about 4.5, and from about 3.5 to about 4.

As used herein the term "ambient room temperature (RT)" refers to a temperature ranging from about 20 to about 25°C.

As used herein the term "optically clear" refers to compositions of the present technology having turbidity that is equal to or less than about 15 NTU, equal to or less than about 10 NTU, equal to or less than about 5 NTU, equal to or less than about 4 NTU, equal to or less than about 3 NTU as measured by the Turbidity Test described in the test protocol hereinbelow (a lower NTU value relates to a composition that is clearer than a composition having a higher NTU value).

In one aspect, the term "optically stable" refers to compositions of the present technology that are "optically clear" at "low pH" values after being maintained at "ambient room temperature" for a period of at least 24 hours, at least about 30 days, at least about 45 days, at least about 50 days.

In one aspect, the term "optically stable" refers to compositions of the present technology that are "optically clear" after being maintained at 45°C for a period of at least about 30 days, at least about 35 days, at least about 4 weeks, at least about 10 weeks, and at least about 14 weeks.

In one aspect, the term "optically stable" refers to compositions of the present technology that are "optically clear" at "low pH" values after being maintained at 45°C for a period of at least about 30 days, at least about 4 weeks, at least about 10 weeks, and at least about 14 weeks.

As used herein, the prefix "(meth)acryl" includes "acryl" as well as "methacryl". For example, the term "(meth)acrylic acid" includes both acrylic acid and methacrylic acid.

Unless otherwise stated, all percentages, parts, and ratios expressed herein are based upon the total weight of the components/monomers contained in the compositions/copolymers of the disclosed technology.

While overlapping weight ranges for the various components, ingredients, and monomers that can be contained in the compositions or copolymers of the disclosed technology have been expressed for selected embodiments and aspects of the disclosed technology, it should be readily apparent that the specific amount of each component in the disclosed compositions/copolymers will be selected from its disclosed range such that the amount of each component/monomer is adjusted such that the sum of all components/monomers in the composition/copolymer will total 100 weight percent. The amounts employed will vary with the purpose and character of the desired product and can be readily determined by one skilled in the art.

The mild, optically stable surfactant compositions containing a non-crosslinked, linear emulsion acrylic copolymer of the disclosed technology may suitably comprise, consist essentially of, or consist of, the components, elements, and process delineations described herein. The disclosed technology illustratively disclosed herein suitably may be practiced in the absence of any element which is not specifically disclosed herein.

Exemplary embodiments in accordance with the present technology are directed to a mild, optically stable surfactant composition comprising a low molecular weight, non-crosslinked, linear acrylic emulsion copolymer that mitigates the ocular and dermal irritation typically associated with surfactant containing compositions without deleteriously affecting the optical clarity properties of the surfactant composition containing a copolymer. Surfactant compositions containing the acrylic copolymers of the disclosed technology are optically stable at low pH and/or prolonged exposure to temperatures of 45°C or more.

In one aspect, the low molecular weight, non-crosslinked, linear acrylic emulsion copolymer utilized in the disclosed technology comprises, consists essentially of, or consists of from 51, 52, 53, 54 or 55 to 75 wt.% of polymerized residues of at least one ethylenically unsaturated $C_3$-$C_6$ carboxylic acid monomer, from 25 to 49 wt.% of polymerized residues of at least one $C_1$-$C_4$ alkyl (meth)acrylate monomer and from 0, 0.05, 0.1, 0.3 or 0.5 to 1 wt.% of a residue of a chain transfer agent (all weights based on the total monomer weight).

Exemplary ethylenically unsaturated $C_3$-$C_6$ carboxylic acid monomers include (meth)acrylic acid, itaconic acid, citraconic acid, maleic acid, maleic anhydride, fumaric acid, crotonic acid, aconitic acid, and mixtures thereof.

Exemplary $C_1$-$C_4$ alkyl (meth)acrylate monomers include methyl (meth)acrylate, ethyl (meth)acrylate, n-butyl (meth)acrylate, t-butyl (meth)acrylate, and mixtures thereof.

When utilized, suitable chain transfer agents include, but are not limited to, thio and disulfide containing compounds, such as $C_1$-$C_{18}$ alkyl mercaptans, mercaptocarboxylic acids, mercaptocarboxylic esters, thioesters, $C_1$-$C_{18}$ alkyl disulfides, aryldisulfides, polyfunctional thiols such as trimethylolpropane-tris-(3-mercaptopropionate), pentaerythritol-tetra-(3-mercaptopropionate), pentaerythritol-tetra-(thioglycolate), and pentaerythritoltetra-(thiolactate), dipentaerythritol-hexa-(thioglycolate), and the like; phosphites and hypophosphites; haloalkyl compounds, such as carbon tetrachloride, bromotrichloromethane, and the like; and catalytic chain transfer agents such as, for example, cobalt complexes (e.g., cobalt (II) chelates).

In one aspect of the present technology, the chain transfer agent is selected from octyl mercaptan, n-dodecyl mercaptan (n-DDM), t-dodecyl mercaptan (t-DDM), hexadecyl mercaptan, octadecyl mercaptan (ODM), isooctyl 3-mercaptopropionate (IMP), butyl 3-mercaptopropionate, 3-mercaptopropionic acid, butyl thioglycolate, isooctyl thioglycolate, and dodecyl thioglycolate.

In one aspect, the chain transfer agent is utilized in an amount ranging from about 0 or 0.05 to about 1 wt.%, from about 0.1 to about 0.75 wt.%, from about 0.3 to about 0.5 wt.% (based on 100 wt. parts of monomer).

In one aspect, the low molecular weight, non-crosslinked, linear acrylic emulsion copolymer comprises, consists essentially of, or consists of from 51 to 75 wt.% of polymerized residues of a monomer selected from (meth)acrylic acid, and from 25 to 49 wt.% of polymerized residues of a monomer selected from ethyl (meth)acrylate, butyl (meth)acrylate, and mixtures thereof, 0, 0.05, 0.1, 0.3 or 0.5 to 1 wt.% of a residue of a chain transfer agent (based on 100 wt. parts of monomer).

In one aspect, the low molecular weight, non-crosslinked, linear emulsion copolymer comprises, consists essentially of, or consists of from 51 to 75 wt.% of polymerized residues of methacrylic acid, and from 25 to 49 wt.% of polymerized residues of ethyl acrylate, and 0 to 0.5 wt.% of a residue of a chain transfer agent (based on 100 wt. parts of monomer).

In one aspect, the low molecular weight, non-crosslinked, linear acrylic emulsion copolymer comprises, consists essentially of, or consists of from 55 to 70 wt.% of polymerized residues of a monomer selected from methacrylic acid, and from 30 to 45 wt.% of polymerized residues of a monomer selected from ethyl acrylate, and 0 to 0.5 wt.% of a residue of a chain transfer agent (based on 100 wt. parts of monomer), wherein the molecular weight of said copolymer rangers from about 50,000 to about 70,000 Daltons ($M_n$).

In one aspect, the low molecular weight, non-crosslinked, linear acrylic emulsion copolymers of the disclosed technology are free of any monomeric repeating unit residues other than the repeating unit residues polymerized from at least one ethylenically unsaturated $C_3$-$C_6$ carboxylic acid monomer and at least one $C_1$-$C_4$ alkyl (meth)acrylate monomer. When an optional chain transfer agent is utilized to prepare the polymers of the disclosed technology, they will contain a residue of said chain transfer agent.

In one aspect, the low molecular weight, non-crosslinked, linear acrylic emulsion copolymer of the disclosed technology has an acid number ≥333 (calculated on the basis of mEq. of KOH/g of polymer). In one aspect, the acid number ranges from 333 to about 475, from about 350 to about 450, and from about 355 to about 430.

The low molecular weight, non-crosslinked, linear acrylic copolymer mitigants of the present technology can be synthesized via free radical polymerization techniques known in the art. In one aspect, emulsion polymerization techniques are used to synthesize the acrylic copolymer mitigants of the disclosed technology. In a typical emulsion polymerization, a mixture of the disclosed monomers is added with mixing agitation to a solution of emulsifying surfactant, such as, for example, an anionic surfactant (e.g., fatty alcohol sulfates or alkyl sulfonates), in a suitable amount of water, in a reactor, to prepare a monomer emulsion. The chain transfer agent if utilized is added to the reaction medium. The emulsion is deoxygenated by any convenient method, such as by sparging with nitrogen, and then a polymerization reaction is initiated by adding a polymerization catalyst (initiator) such as sodium persulfate, or any other suitable addition polymerization catalyst, as is well known in the emulsion polymerization art. The polymerization medium is agitated until the polymerization is complete, typically for a time in the range of about 4 to about 16 hours. The monomer emulsion can be heated to a temperature in the range of about 70 to about 95°C prior to addition of the initiator, if desired. Unreacted monomer can be eliminated by addition of more catalyst, as is well known in the emulsion polymerization art. The resulting polymer emulsion product can then be discharged from the reactor and packaged for storage or use. Optionally, the pH or other physical and chemical characteristics of the emulsion can be adjusted prior to discharge from the reactor. In one aspect, the product emulsion has a total polymer solids content in the range of about 10 to about 50 wt.%. In one aspect, the polymer solids of the emulsion product is in the range of about 15 to about 45 wt.%. In one aspect, the polymer solids of the emulsion product ranges from about 25 to 35 wt.%.

Suitable surfactants for facilitating emulsion polymerizations include nonionic, anionic, amphoteric, cationic surfactants, and mixtures thereof. Most commonly, nonionic surfactants, anionic surfactants, and mixtures thereof are utilized in the emulsion polymerization.

Nonionic surfactants suitable for facilitating emulsion polymerizations are well known in the polymer art, and include, without limitation, linear or branched alcohol ethoxylates, $C_8$ to $C_{12}$ alkylphenol alkoxylates, such as octylphenol ethoxylates, polyoxyethylene polyoxypropylene block copolymers, and the like. Other useful nonionic surfactants include $C_8$ to $C_{22}$ fatty acid esters of polyoxyethylene glycol, mono and diglycerides, sorbitan esters and ethoxylated sorbitan esters, $C_8$ to $C_{22}$ fatty acid glycol esters, block copolymers of ethylene oxide and propylene oxide having an HLB value of greater than about 15, ethoxylated octylphenols, and combinations thereof.

Exemplary alkylphenol alkoxylate surfactants include an octylphenol sold under the trade name IGEPAL® CA-897 by Rhodia, Inc. Exemplary linear alcohol alkoxylates include polyethylene glycol ethers of cetearyl alcohol (a mixture of cetyl and stearyl alcohols) sold under the trade names PLURAFAC® C-17, PLURAFAC® A-38 and PLURAFAC® A-39 by BASF Corp. Exemplary polyoxyethylene polyoxypropylene block copolymers include copolymers sold under the trade names PLURONIC® F127, and PLURONIC® L35 by BASF Corp.

Other Exemplary nonionic surfactants include Ethoxylated (50) linear fatty alcohols such as DISPONIL® A 5060 (Cognis), branched alkyl ethoxylates such as GENAPOL® X 1005 (Clariant Corp.), secondary $C_{12}$ to $C_{14}$ alcohol ethoxylates such as TERGITOL® S15-30 and S15-40 (Dow Chemical Co.), ethoxylated octylphenol-based surfactants such as TRITON® X-305, X-405 and X-705 (Dow Chemical Co.), IGEPAL® CA 407, 887, and 897 (Rhodia, Inc.), ICONOL® OP 3070 and 4070 (BASF Corp.), SYNPERONIC® OP 30 and 40 (Uniqema), block copolymers of ethylene oxide and propylene oxide such as PLURONIC® L35 and F127 (BASF Corp.), and secondary $C_{11}$ alcohol ethoxylates such as EMULSOGEN® EPN 407 (Clariant Corp.). Numerous other suppliers are found in the trade literature.

Anionic surfactants suitable for facilitating emulsion polymerizations are well known in the polymer art, and include sodium lauryl sulfate, sodium dodecyl benzene sulfonate, sodium dioctyl sulfosuccinate, sodium di-sec-butyl naphthylene sulfonate, disodium dodecyl diphenyl ether sulfonate, and disodium n-octadecyl sulfosuccinate, and the like.

Polymeric stabilizers (also known as protective colloids) can be utilized in the emulsion polymerization process. The polymeric stabilizers are water-soluble polymers, including, for example, synthetic polymers, such as polyvinyl alcohol, partially hydrolyzed polyvinyl acetate, polyvinylpyrrolidone, polyacrylamide, polymethacrylamide, carboxylate-functional addition polymers, polyalkyl vinyl ethers and the like; water-soluble natural polymers, such as gelatin, pectins, alginates, casein, starch, and the like; and modified natural polymers, such as methylcellulose, hydroxypropylcellulose, carboxymethylcellulose, allyl modified hydroxyethylcellulose, and the like. In some cases, it can be of advantage to use mixtures of a synthetic and a natural protective colloid, for example, a mixture of polyvinyl alcohol and casein. Further suitable natural polymers are mixed ethers such as methylhydroxyethylcellulose and carboxymethylmethylcellulose. Polymeric stabilizers can be utilized in amounts up to about 2 wt.% based on the total emulsion weight. When utilized, a polymeric stabilizer can be included in an amount in the range of about 0.0001 to about 2 wt.% in one aspect, and in another aspect from about 0.01 wt.% to about 1.0 wt.%.

Exemplary free radical initiators include, without limitation, the water-soluble inorganic persulfate compounds, such as ammonium persulfate, potassium persulfate, and sodium persulfate; peroxides such as hydrogen peroxide, benzoyl peroxide, acetyl peroxide, and lauryl peroxide; organic hydroperoxides, such as cumene hydroperoxide and t-butyl hydroperoxide; organic peracids, such as peracetic acid; and oil soluble, free radical producing agents, such as 2,2'-azobisisobutyronitrile, and the like, and mixtures thereof. Peroxides and peracids can optionally be activated with reducing agents, such as sodium bisulfite or ascorbic acid, transition metals, hydrazine, sulfinic acid derivatives such as Bruggolite® FF6 which contains a mixture of the disodium salt of 2-hydroxy-2-sulfinatoacetate, the disodium salt of 2-hydroxy-2-sulfonatoacetate and sodium sulfite (commercially available from Bruggemann Chemical US), and the like. Other free-radical polymerization initiators include water soluble azo polymerization initiators, such as 2,2'-azobis(tert-alkyl) compounds having a water solubilizing substituent on the alkyl group. Additional azo polymerization catalysts include the VAZO® free-radical polymerization initiators, available from DuPont, such as VAZO® 44 (2,2'-azobis(2-(4,5-dihydroimidazolyl)propane), VAZO® 56 (2,2'-azobis(2-methylpropionamidine) dihydrochloride), and VAZO® 68 (4,4'-azobis(4-cyanovaleric acid)).

Optionally, other emulsion polymerization additives, which are well known in the emulsion polymerization art, such as solvents, buffering agents, chelating agents, inorganic electrolytes, chain terminators, and pH adjusting agents can be included in the polymerization system.

A general emulsion polymerization procedure for the preparation of the non-crosslinked, linear acrylic copolymer mitigants of the present technology is exemplified herein.

In one aspect, the low molecular weight, non-crosslinked, linear copolymeric irritation mitigants of the disclosed technology have a viscosity of 500 mPa·s or less (Brookfield RVT, 20 rpm, spindle No. 1) at a 5 wt.% polymer solids concentration in deionized water and neutralized to pH 7 with an 18 wt.% NaOH solution. In another aspect, the viscosity ranges from about 1 to about 500 mPa·s, from about 10 to about 250 mPa·s in a further aspect, and from about 15 to about 150 mPa·s in a still further aspect.

The low molecular weight, non-crosslinked, linear acrylic copolymers can be utilized in surfactant compositions in the un-neutralized state or can be neutralized to a desired degree of neutralization with a suitable alkaline neutralizing agent. The amount of alkaline neutralizing agent employed to obtain a desired degree of neutralization is calculated on the basis of the acid number of the polymer. Exemplary neutralizing agents include sodium hydroxide, potassium hydroxide, triethanolamine, fatty acid amines, and the like. Alternatively, other alkaline materials can be used, such as, for example, pre-neutralized surfactants. In one aspect, the degree of polymer neutralization is 100% or less, in another aspect the degree of polymer neutralization is 80% or less, in still another aspect the degree of polymer neutralization is 60% or less. In a further aspect, the degree of neutralization is 50% or less. In a still further aspect, the degree of neutralization is 40, 30, and 20% or less. In another aspect, the degree of polymer neutralization can range from about 0% or 1% to about 100%, in still another aspect from about 0% or 1% to about 80%, in a further aspect from about 0% or 1% to about 60%, in a still further aspect from about 5% to about 40%, and in another aspect from about 10% to about 35%, and in a further aspect from about 15% to about 30%.

In one aspect, the low molecular weight, non-crosslinked, linear acrylic copolymer of the disclosed technology is added to a composition comprising at least one surfactant. The composition can be neutralized with an alkaline neutralization agent (described above) to achieve a final pH value ranging from about 6 to about 8 in one aspect, from about 6.5 to about 7.5 in another aspect and from about 6.7 to about 7.3 in a further aspect. The alkaline neutralization agent can be added to the surfactant composition at any stage in the formulation process as long as the desired final pH of the formulation is attained and the desired formulation properties are not deleteriously impacted.

In one aspect, if a low pH formulation is desired the low molecular weight, non-crosslinked, linear acrylic copolymer of the disclosed technology can be added to the surfactant composition and neutralized as described immediately above to a pH value ranging from about from about 6 to about 8 in one aspect, from about 6.5 to about 7.5 in another aspect and from about 6.7 to about 7.3 in a further aspect, and about 7 in a still further aspect, followed by back-acid titration with a food grade preservative acid to a pH of ≤5.5 in one aspect, from about 2 to about 5.5, from about 2.5 to about 5, from about 3 to about 4.5, and from about 3.5 to about 4 in further aspects. Without wishing to be bound by theory of operation, it is believed that neutralizing the acid groups on the polymer backbone "opens" the polymer chains from an entangled state to a less entangled state through ionic repulsion, thus permitting more interaction of the polymer chains with the surfactant.

In one aspect, the food grade acid preservative can be added to the surfactant composition containing without first adding an alkaline neutralization agent as described immediately above so long as the pH of the composition is adjusted to a value of ≤5.5 in one aspect, from about 2 to about 5.5, from about 2.5 to about 5, from about 3 to about 4.5, and from about 3.5 to about 4 in further aspects. In this embodiment, the pH of the surfactant composition can be adjusted at any stage during the formulation process as long as the desired final pH of the formulation is attained and the desired formulation properties are not deleteriously impacted.

Exemplary food based acid preservatives that can be used in the compositions of the disclosed technology are, but are not limited to, benzoic acid, acetic acid, propionic acid, sorbic acid, salicylic acid, lactic acid, citric acid, furmaric acid, malic acid, the calcium, potassium and sodium salts thereof, and mixtures thereof. Exemplary salts include, but are not limited to sodium benzoate, potassium sorbate, sodium propionate, calcium dipropionate, and mixtures thereof. When utilizing the salts of the foregoing food grad acids, it is more effective to utilize them at or below their pKa. If necessary, the pH of the surfactant composition can be adjusted to a range to facilitate the use of the food grade acid salts at or below their pKa with food grade acids that are in their free (protonated) form.

In one aspect, the food grade acid preservatives and/or their salts can be present from about 0.01% to about 3% by weight, from about 0.1% to about 1% by weight in another aspect, and from about 0.3% to about 1% by weight in a further aspects, based on the total weight of the composition of the present technology. However, the amount of food grade acid preservative utilized in the composition must achieve the desired value(s) within the low pH range.

According to certain aspects of the present technology, low molecular weight, non-crosslinked, linear copolymeric irritation mitigant is combined with anionic detersive surfactants typically contained in personal care, animal care and household care cleansers and cleaners. Exemplary personal care cleansers include but are not limited to shampoos (e.g., 2-in-1 shampoos, conditioning shampoos, bodifying shampoos; moisturizing shampoos, temporary hair color shampoos, 3-in-1 shampoos, anti-dandruff shampoos, hair color maintenance shampoos, acid (neutralizing) shampoos, salicylic acid shampoos, medicated shampoos, baby shampoos, and the like), and skin and body cleansers (e.g., moisturizing body washes, antibacterial body washes; bath gels, shower gels, liquid hand soaps, bar soaps, body scrubs, bubble baths, facial scrubs, foot scrubs, and the like). Exemplary household care cleaners include but are not limited to home care and industrial and institutional applications (e.g., laundry detergents, dishwashing detergents (automatic and manual), hard surface cleaners, heavy duty hand soaps, cleaners and sanitizers, automotive cleaners, and the like). Exemplary pet and animal care cleansers include but are not limited to shampoos, medicated shampoos, conditioning shampoos (e.g., detangling, antistatic, grooming), and foaming shampoos.

The irritation mitigated compositions contain various surfactants such as anionic, amphoteric, zwitterionic, nonionic, cationic, or combinations thereof.

The anionic surfactant can be any of the anionic surfactants known or previously used in the art of aqueous surfactant compositions. Suitable anionic surfactants include but are not limited to alkyl sulfates, alkyl ether sulfates, alkyl ether sulfonates, alkaryl sulfonates, alkyl succinates, alkyl sulfosuccinates, N-alkoyl sarcosinates, alkyl phosphates, alkyl ether phosphates, alkyl ether carboxylates, alkylamino acids, alkyl peptides, alkoyl taurates, carboxylic acids, acyl and alkyl glutamates, alkyl isethionates, and alpha-olefin sulfonates, especially their sodium, potassium, magnesium, ammonium and mono-, di- and triethanolamine salts. The alkyl groups generally contain from 6 to 26 carbon atoms and can be saturated or unsaturated. The aryl groups generally contain 6 to 14 carbon atoms. The alkyl ether sulfates, alkyl ether sulfonates, alkyl ether phosphates and alkyl ether carboxylates can contain from 1 to 25 ethylene oxide and/or propylene oxide units per molecule in one aspect, and from 1 to 10 ethylene oxide and/or propylene oxide units per molecule in another aspect. In one aspect, the alkaryl sulfonate is alkyl benzene sulfonate and salts thereof (e.g., sodium, potassium, magnesium, etc.) wherein the alkyl group contains 8 to 16 carbon atoms. In another aspect, the alkaryl sulfonate is dodecyl benzene sulfonate and salts thereof (e.g., sodium, potassium, magnesium, etc.). Other surfactants are disclosed in U.S. Patent No. 6,051,541 which is herein incorporated by reference.

Examples of suitable anionic surfactants include sodium and ammonium laureth sulfate and sodium trideceth sulfate (each ethoxylated with 1 to 4 moles of ethylene oxide), sodium, ammonium, and triethanolamine lauryl sulfate, disodium laureth sulfosuccinate, sodium cocoyl isethionate, sodium $C_{12}$ to $C_{14}$ olefin sulfonate, sodium laureth-6 carboxylate, sodium laureth-13 carboxylate, sodium $C_{12}$ to $C_{15}$ pareth sulfate, sodium methyl cocoyl taurate, sodium dodecylbenzene sulfonate, sodium cocoyl sarcosinate, triethanolamine monolauryl phosphate, and fatty acid soaps.

The nonionic surfactant can be any of the nonionic surfactants known or previously used in the art of aqueous surfactant compositions. Suitable nonionic surfactants include but are not limited to aliphatic $C_6$ to $C_{18}$ primary or secondary linear or branched chain acids, alcohols or phenols, linear alcohol and alkyl phenol alkoxylates (especially ethoxylates and mixed ethoxy/propoxy), block alkylene oxide condensate of alkyl phenols, alkylene oxide condensates of alkanols, ethylene oxide/propylene oxide block copolymers, semi-polar nonionics (e.g., amine oxides and phosphine oxides), as well as alkyl amine oxides. Other suitable nonionics include mono or di alkyl alkanolamides and alkyl polysaccharides, sorbitan fatty acid esters, polyoxyethylene sorbitan fatty acid esters, polyoxyethylene sorbitol esters, and polyoxyethylene acids. Examples of suitable nonionic surfactants include coco mono- or diethanolamide, coco diglucoside, alkyl polyglucoside, cocamidopropyl and lauramine oxide, polysorbate 20, ethoxylated linear alcohols, cetearyl alcohol, lanolin alcohol, stearic acid, glyceryl stearate, PEG-150 distearate, PEG-100 stearate, PEG-80 sorbitan laurate, and oleth 20.

In one aspect, the nonionic surfactant is an alcohol alkoxylate wherein the alcohol residue contains 8 to 18 carbon atoms and the number of moles of alkylene oxide is from about 3 to about 12. The alkylene oxide moiety is selected from ethylene oxide, propylene oxide and combinations thereof. In another aspect, the alcohol alkoxylate can be derived from a fatty alcohol containing 8 to 15 carbon atoms and can contain from 5 to 10 alkoxy groups (e.g. ethylene oxide, propylene oxide, and combinations thereof). Exemplary nonionic alcohol alkoxylate surfactants in which the alcohol residue contains 12 to 15 carbon atoms and contain about 7 ethylene oxide groups are available under the Tomadol® (e.g., product designation 25-7) and Neodol® (e.g., product designation 25-7) trade names from Tomah Products, Inc. and Shell Chemicals, respectively.

Another commercially available alcohol alkoxylate surfactant is sold under the Plurafac® trade name from BASF. The Plurafac surfactants are reaction products of a higher linear alcohol and a mixture of ethylene and propylene oxides, containing a mixed chain of ethylene oxide and propylene oxide, terminated by a hydroxyl group. Examples include $C_{13}$ to $C_{15}$ fatty alcohols condensed with 6 moles ethylene oxide and 3 moles propylene oxide, $C_{13}$ to $C_{15}$ fatty alcohols condensed with 7 moles propylene oxide and 4 moles ethylene oxide, and $C_{13}$ to $C_{15}$ fatty alcohols condensed with 5 moles propylene oxide and 10 moles ethylene oxide.

Another commercially suitable nonionic surfactant is available from Shell Chemicals under the Dobanol™ trade name (product designations 91-5 and 25-7). Product designation 91-5 is an ethoxylated $C_9$ to $C_{11}$ fatty alcohol with an average of 5 moles ethylene oxide and product designation 25-7 is an ethoxylated $C_{12}$ to $C_{15}$ fatty alcohol with an average of 7 moles ethylene oxide per mole of fatty alcohol.

Amphoteric and zwitterionic surfactants are those compounds which have the capacity of behaving either as an acid or a base. These surfactants can be any of the surfactants known or previously used in the art of aqueous surfactant compositions. Suitable materials include but are not limited to alkyl betaines, alkyl amidopropyl betaines, alkyl sulphobetaines, alkylglycinates, alkylcarboxyglycinates, alkylamphopropionates, alkylamphoglycinates, alkylamidopropyl hydroxysultaines, acyl taurates and acyl glutamates wherein the alkyl and acyl groups have from 8 to 18 carbon atoms. Examples include cocamidopropyl betaine, sodium cocoamphoacetate, cocamidopropyl hydroxysultaine, lauroamphoglycinate, and sodium cocamphopropionate.

The cationic surfactants can be any of the cationic surfactants known or previously used in the art of aqueous surfactant compositions. Suitable cationic surfactants include but are not limited to alkyl amines, alkyl imidazolines, ethoxylated amines, quaternary compounds, and quaternized esters. In addition, alkyl amine oxides can behave as a cationic surfactant at a low pH. Examples include lauramine oxide, dicetyldimonium chloride, cetrimonium chloride.

Other surfactants which can be utilized in the present technology are set forth in more detail in WO 99/21530, U.S. Patent No. 3,929,678, U.S. Patent No. 4,565,647, U.S. Patent No. 5,456,849, U.S. Patent No. 5,720,964, U.S. Patent No. 5,858,948, and U.S. Patent No. 7,115,550, which are herein incorporated by reference. Other suitable surfactants are described in McCutcheon's Emulsifiers and Detergents (North American and International Editions, by Schwartz, Perry and Berch).

In one aspect, the low molecular weight non-crosslinked, linear acrylic copolymer of the present technology is utilized in any amount that is sufficient to increase the critical micelle concentration (CMC) of a surfactant containing composition in comparison to a comparable surfactant composition which is free of the non-crosslinked, linear acrylic copolymer. In another aspect of the present technology, the non-crosslinked, linear acrylic copolymer is utilized in any amount effective to mitigate ocular and/or dermal irritation typically associated with surfactant compositions. The CMC value of a surfactant containing composition can readily be determined as disclosed in International Patent Application No. WO 2005/023870 and U.S. Patent Nos. 7,084,104 and 7,098,180 as well as exemplified in the examples which follow.

Irritation elicited by a surfactant containing composition can be measured by the Trans-Epithelial Permeability (TEP) Test as set forth in Invittox Protocol No. 86 (May 1994). As disclosed in WO 2005/023870, supra, Trans-Epithelial Permeability (TEP) values have a direct correlation to the ocular and/or dermal irritation associated with a particular surfactant composition. Higher TEP values are indicative of milder compositions as compared to compositions having lower TEP values.

The amount of low molecular weight, non-crosslinked, linear acrylic copolymer utilized in surfactant containing compositions, such as, for example, personal care cleansing, animal and pet care cleansing, household care cleaning, and industrial and institutional cleaning compositions can range from about 0.05 wt.% to about 10 wt.% (active polymer solids) in one aspect, from about 0.1 wt.% to 6 wt.%, from about 0.5 wt.% to 4 wt.%, and from about 1 wt.% to about 3 wt.% in further aspects, based on the total weight of the total composition.

In one aspect, the surfactant(s) utilized in the surfactant containing composition can be employed in amounts typically utilized in personal care cleansing and animal and pet care cleansing, household care cleaning, and industrial and institutional cleaning compositions. In another aspect, the amount of surfactant(s) can range from about 0.1 wt.% to about 50 wt.%, based on the total weight of the surfactant containing composition. In a further aspect, the amount of surfactant(s) ranges from about 0.5 wt.% to about 45 wt.%, from about 1 wt.% to about 30 wt.% in a still further aspect, from about 5 wt.% to about 25 wt.%, and from about 10 wt.% to about 20 wt.% (all percentages based on the weight of the total surfactant containing composition). One advantage of utilizing the irritation mitigating polymers of the present technology is that the polymers permit higher amounts of surfactant to be employed in cleansing and cleaning compositions which in turn enhances the detersive properties of such compositions without adversely affecting the rheology profile. Accordingly, higher amounts of surfactant than typically utilized above can be employed.

In one aspect, the surfactant is selected from a combination of an anionic surfactant and an amphoteric surfactant. In one aspect, the ratio of anionic surfactant to amphoteric surfactant (active material) is 10:1 to about 2:1 in one aspect, and 9:1, 8:1, 7:1 6:1, 5:1, 4.5:1, 4:1, or 3:1 in another aspect.

Water is utilized as a diluent in the mitigated surfactant compositions of the present technology. In one aspect, the amount of water can range from about 5 wt.% to about 95 wt.% of the weight of the total surfactant containing composition. In another aspect, the amount of water can range from about 10 wt.% to about 90 wt.%, from about 20 wt.% to about 80 wt.% in a further aspect, and from about 30 wt.% to about 75 wt.% in a still further aspect, based on the total weight of the surfactant containing composition.

The surfactant compositions of the present technology can contain one or more of a wide variety of components well known to those skilled in the art, such as chelators, humectant skin or hair conditioners, lubricants, moisture barriers/emollients, opacifiers, preservatives, spreading aids, conditioning polymers, vitamins, viscosity adjusters, viscosity modifiers/emulsifiers, suspended beads, enzymes, builders, electrolytes (e.g., NaCl), buffers, hydrotropes (e.g., ethanol, sodium xylene sulfonate, and sodium cumene sulfonate), inorganics (e.g., clay, bentonite, kaolin), soil releasing agents, color additives, as well as the numerous other optional components for enhancing and maintaining the properties of the personal care compositions. Such components are also described in detail in well known sources such as Mitchell C. Schlossman, The Chemistry and Manufacture of Cosmetics, Volumes I and II, Allured Publishing Corporation, 2000.

Suitable chelators include EDTA (ethylene diamine tetraacetic acid) and salts thereof such as disodium EDTA and tetrasodium ETDA, citric acid and salts thereof, cyclodextrins, and the like, and mixtures thereof. Such suitable chelators typically comprise from about 0.001 wt.% to about 3 wt.% in one aspect, from about 0.01 wt.% to about 2 wt.% in another aspect, and from about 0.01 wt.% to about 1 wt.% in a further aspect of the present technology based on the total weight of the surfactant containing composition.

Suitable humectant skin and/or hair conditioners include allantoin; pyrrolidonecarboxylic acid and its salts; hyaluronic acid and its salts; sorbic acid and its salts, salicylic acid and its salts; urea; lysine, arginine, cystine, guanidine, and other amino acids; polyhydroxy alcohols such as glycerin, propylene glycol, hexylene glycol, hexanetriol, ethoxydiglycol, dimethicone copolyol, and sorbitol, and the esters thereof; polyethylene glycol; glycolic acid and glycolate salts (e.g., ammonium and quaternary alkyl ammonium); lactic acid and lactate salts (e.g., ammonium and quaternary alkyl ammonium); sugars and starches; sugar and starch derivatives (e.g., alkoxylated glucose); D-panthenol; lactamide monoethanolamine; acetamide monoethanolamine; and the like, and mixtures thereof. Preferred humectants include the $C_3$ to $C_6$ diols and triols, such as glycerin, propylene glycol, hexylene glycol, hexanetriol, and the like, and mixtures thereof. Such suitable humectants typically comprise from about 1 wt.% to about 10 wt.% in one aspect, from about 2 wt.% to about 8 wt.% in another aspect, and from about 3 wt.% to about 5 wt.% in a further aspect of the present technology, based on the total weight of the surfactant containing composition.

Suitable lubricants include volatile silicones, such as cyclic or linear polydimethylsiloxanes, and the like. The number of silicon atoms in cyclic silicones preferably is from about 3 to about 7 and more preferably 4 or 5. Exemplary volatile silicones, both cyclic and linear, are available from Dow Corning Corporation as Dow Corning 344, 345 and 200 fluids. The linear volatile silicones typically have viscosities of less than about 5 cP at 25°C, while the cyclic volatile silicones typically have viscosities of less than about 10 cP at 25°C. "Volatile" means that the silicone has a measurable vapor pressure. A description of volatile silicones can be found in Todd and Byers, "Volatile Silicone Fluids for Cosmetics", Cosmetics and Toiletries, Vol. 91, January 1976, pp. 27-32. Other suitable lubricants include polydimethylsiloxane gums, aminosilicones, phenylsilicones, polydimethyl siloxane, polydiethylsiloxane, polymethylphenylsiloxane, polydimethylsiloxane gums, polyphenyl methyl siloxane gums, amodimethicone, trimethylsiloxyamodimethicone, diphenyl-dimethyl polysiloxane gums, and the like. Mixtures of lubricants can also be used. Such suitable lubricants typically comprise from about 0.10 wt.% to about 15 wt.% in one aspect, from about 0.1 wt.% to about 10 wt.% in another aspect, and from about 0.5 wt.% to about 5 wt.% in a further aspect of the present technology, based on the total weight of the surfactant containing composition.

Suitable moisture barriers and or emollients include mineral oil; stearic acid; fatty alcohols such as cetyl alcohol, cetearyl alcohol, myristyl alcohol, behenyl alcohol, and lauryl alcohol; cetyl acetate in acetylated lanolin alcohol, isostearyl benzoate, dicaprylyl maleate, caprylic and capric triglyceride; petrolatum, lanolin, coco butter, Avena sativa (oat) kernel oil, shea butter, beeswax and esters there of; ethoxylated fatty alcohol esters such as ceteareth-20, oleth-5, and ceteth-5; avocado oil or glycerides; sesame oil or glycerides; safflower oil or glycerides; sunflower oil or glycerides; botanical seed oils; volatile silicone oils; non-volatile emollients, and the like, and mixtures thereof. Suitable non-volatile emollients include fatty acid and fatty alcohol esters, highly branched hydrocarbons, and the like, and mixtures thereof. Such fatty acid and fatty alcohol esters include decyl oleate, butyl stearate, myristyl myristate, octyldodecyl stearoylstearate, octylhydroxystearate, di-isopropyl adipate, isopropyl myristate, isopropyl palmitate, ethyl hexyl palmitate, isodecyl neopentanoate $C_{12}$ to $C_{15}$ alcohol benzoate, diethyl hexyl maleate, PPG-14 butyl ether and PPG-2 myristyl ether propionate, cetearyl octanoate, and the like, and mixtures thereof. Suitable highly branched hydrocarbons include isohexadecane and the like, and mixtures thereof. Such suitable moisture barriers and/or emollients, alone or in combination, typically comprise from about 1 wt.% to about 20 wt.% in one aspect, from about 2 wt.% to about 15 wt.% in another aspect, and from about 3 wt.% to about 10 wt.% in a further aspect of the present technology, based on the total weight of the surfactant containing composition.

Suitable opacifiers include glycol fatty acid esters; alkoxylated fatty acid esters; polymeric opacifiers, fatty acid alcohols; hydrogenated fatty acids, waxes and oils; kaolin; magnesium silicate; titanium dioxide; silica; and the like, and mixtures thereof. Such suitable opacifiers typically comprise from about 0.1 wt.% to about 8 wt.% in one aspect, from about 0.5 wt.% to about 6 wt.% in another aspect, and from about 1 wt.% to about 5 wt.% in a further aspect of the present technology, based on the total weight of the surfactant containing composition.

When utilizing conventional preservatives, suitable preservatives include polymethoxy bicyclic oxazolidine, methylparaben, propylparaben, ethylparaben, butylparaben, benzyltriazole, DMDM hydantoin (also known as 1,3-dimethyl-5,5-dimethyl hydantoin), imidazolidinyl urea, phenoxyethanol, phenoxyethylparaben, methylisothiazolinone, methylchloroisothiazolinone, benzoisothiazolinone, triclosan, quaternium-15, salicylic acid salts, and the like, and mixtures thereof. Such suitable preservatives typically comprise about 0.01 wt.% to about 1.5 wt.% in one aspect, from about 0.1 wt.% to about 1 wt.% in another aspect, and from about 0.3 wt.% to about 1 wt.% in a further aspect, based on the total weight of the surfactant containing composition. The conventional preservatives can be utilizing in lieu of or in combination with the food grade acid preservatives mentioned above.

Suitable spreading aids include hydroxypropyl methylcellulose, hydrophobically modified cellulosics, xanthan gum, cassia gum, guar gum, locust bean gum, dimethicone copolyols of various degrees of alkoxylation, boron nitride, talc, and the like, and mixtures thereof. Such suitable spreading aids typically comprise about 0.01 wt.% to about 5 wt.% in one aspect, from about 0.1 wt.% to about 3 wt.% in another aspect, and from about 0.1 wt.% to about 2.0 wt.% in a further aspect of the invention, based on the total weight of the surfactant containing composition.

Suitable conditioning polymers include quaternized polygalactomannans such as cationic guar, cationic cassia, cationic locust bean, quaternized cellulosics, polyquaternium-4, polyquaternium-5 polyquaternium-6, polyquaternium-7, polyquaternium-10, polyquaternium-11, polyquaternium-39, polyquaternium-44, polyquaternium-47, polyquaternium-53, and the like, and mixtures thereof. Such suitable conditioning agents typically comprise about 0.01 wt.% to about 3 wt.% in one aspect, from about 0.1 wt.% to about 2 wt.% in another aspect, and from about 0.1 wt.% to about 1 wt.% in a further aspect of the invention, based on total weight of the surfactant containing composition.

Suitable vitamins include vitamin A, vitamin B, biotin, pantothenic acid, vitamin C, vitamin D, vitamin E, tocopherol acetate, retinyl palmitate, magnesium ascorbyl phosphate, and the like, and derivatives and mixtures thereof.

Suitable viscosity adjusters include isopropyl alcohol, ethanol, sorbitol, propylene glycol, diethylene glycol, triethylene glycol, dimethyl ether, butylene glycol, and the like, and mixtures thereof. Such suitable viscosity adjusters typically comprise from about 0.1 wt.% to about 60 wt.% in one aspect, from about 1 wt.% to about 40 wt.% in another aspect, and from about 5 wt.% to about 20 wt.% in a further aspect of the invention based on the total weight of the surfactant containing compositions.

Suitable viscosity modifiers/emulsifiers include natural, semisynthetic, and synthetic polymers. Examples of natural and modified natural polymers include xanthan gums, cellulosics, modified cellulosics, starches, polysaccharides, and the like. Examples of synthetic polymers include crosslinked polyacrylates, alkali swellable emulsion acrylate copolymers, hydrophobically modified alkali swellable copolymers, hydrophobically modified non-ionic polyurethanes, and the like. Mixtures can also be used. Such suitable viscosity modifiers/emulsifiers, alone or in combination, typically comprise from about 0.1 wt.% to about 5 wt.% in one aspect, from about 0.3 wt.% to about 3 wt.% in another aspect, and from about 0.5 wt.% to about 2 wt.% in still another aspect of the invention, based on the total weight of the surfactant containing compositions.

When used in conjunction with a suspending agent, the surfactant containing composition can contain from about 0.1 wt.% to about 10 wt.% based on the total weight of the composition of a cosmetic bead component suspended in the composition. Cosmetic beads can be included for aesthetic appearance or can function as micro- and macroencapsulants in the delivery of beneficial agents to the skin. Exemplary bead components include but are not limited to microsponges, gelatin beads; alginate beads; expanded polystyrene beads; jojoba beads; polyethylene beads; Unispheres® cosmetic beads (Induchem), such as for example, product designations YE-501 and UEA-509; Lipopearls™ vitamin E encapsulated in gelatin beads (Lipo Technologies Inc.); and Confetti™ (United Guardian Company). A suitable suspending agent includes a crosslinked acrylic copolymer rheology modifier such as Carbopol® Aqua SF-1, Carbopol® Aqua SF-2 available from Noveon Consumer Specialties of Lubrizol Advanced Materials, Inc. Such rheology modifiers can be employed in a range of from about 1.5 wt.% to about 5 wt.% (polymer solids), based on the weight of the surfactant containing composition.

Other optional components can be used in order to maintain and enhance the properties of personal care compositions. Such optional components include various solvents, propellants, combing aids, pearlizing agents, botanical extracts, antioxidants, antistatic agents, anticorrosion agents, agents suitable for product aesthetics, such as fragrances, perfumes, pigments, dyes, and colorings, and the like.

It is also to be recognized that the choice and amount of ingredients in surfactant containing compositions including the polymer irritation mitigants of the present technology will vary depending on the intended product and its function, as is well known to those skilled in the formulation arts. An extensive listing of substances and their conventional functions and product categories appears in the INCI Dictionary, generally, and in Vol. 2, Sections 4 and 5 of the Seventh Edition.

The polymers of the disclosed technology and the surfactant(s) may be combined according to the present invention via any conventional methods of combining two or more fluids. For example, one or more compositions comprising, consisting essentially of, or consisting of at least one disclosed polymeric material and one or more compositions comprising, consisting essentially of, or consisting of at least one anionic and/or amphoteric surfactant may be combined by pouring, mixing, adding dropwise, pipetting, pumping, and the like, one of the compositions comprising polymeric material or surfactant into or with the other in any order using any conventional equipment such as a mechanically stirred propeller, paddle, and the like. According to certain aspects, for example, the combining step comprises combining a composition comprising anionic and/or amphoteric surfactant into or with a composition comprising the disclosed polymeric material. According to certain other aspects, for example, the combining step comprises combining a composition comprising the disclosed polymeric material into or with a composition comprising the anionic and/or amphoteric surfactant.

The reduced irritation surfactant compositions produced, as well as any of the compositions comprising the disclosed polymeric material or anionic and/or amphoteric surfactant that are combined in the combining step according to the present methods may further comprise any of a variety of other components nonexclusively including one or more nonionic and/or cationic surfactants, pearlescent or opacifying agents, thickening agents, conditioners, humectants, chelating agents, and additives which enhance the appearance, feel and fragrance of the compositions, such as colorants, fragrances, preservatives (conventional and food grade acids), pH adjusting agents, and the like.

The following examples further describe and demonstrate embodiments within the scope of the present technology. These examples are presented solely for the purpose of illustration, and are not to be construed as limitations of the present technology since many variations thereof are possible without departing from the spirit and scope thereof. Unless otherwise specified weight percent (wt.%) is given in weight percent based on the weight of the total composition.

**Methods Description**

**Turbidity**

When reported, the turbidity of a surfactant containing composition was determined in Nephelometric Turbidity Units (NTU) employing a nephelometric turbidity meter (Mircro 100 Turbidimeter, HF Scientific, Inc.) with distilled water (NTU = 0) as the standard. Six dram screw cap vials (70 mm x 25 mm) are filled almost to the top with test sample and centrifuged at 100 rpm until all bubbles are removed. Upon centrifugation each sample vial is wiped with tissue paper to remove any smudges before placement in the turbidity meter. The sample is placed in the turbidity meter and a reading is taken. Once the reading stabilizes the NTU value is recorded. The vial is given one-quarter turn and another reading is taken and recorded. This is repeated until four readings are taken. The lowest of the four readings is reported as the turbidity value. Compositions having an NTU value of about 90 or greater were judged turbid. All measurements unless otherwise specified are conducted at ambient room temperature (20-25°C).

**Viscosity**

Brookfield rotating spindle method: The viscosity of each polymer containing composition is measured as mPa·s, employing a Brookfield rotating spindle viscometer, Model RVT (Brookfield Engineering Laboratories, Inc.), at about 20 revolutions per minute (rpm), at ambient room temperature of about 20 to 25°C (hereafter referred to as viscosity). Appropriate spindle sizes are set forth in the examples.

**Yield Value**

Yield °Value, also referred to as Yield Stress, is defined as the initial resistance to flow under stress. It is measured by the Brookfield Yield Value (BYV) Extrapolation Method using a Brookfield viscometer (Model RVT). The Brookfield viscometer is used to measure the torque necessary to rotate a spindle through a liquid sample at speeds of 0.5 to 100 rpm. Multiplying the torque reading by the appropriate constant for the spindle and speed gives the apparent viscosity. Yield Value is an extrapolation of measured values to a shear rate of zero. The BYV is calculated by the following equation:

$$BYV,\ dyn/cm^2 = (\eta_{\alpha 1} - \eta_{\alpha 2})/100$$

where $\eta_{\alpha 1}$ and $\eta_{\alpha 2}$ = apparent viscosities obtained at two different spindle speeds (0.5 rpm and 1.0 rpm, respectively). These techniques and the usefulness of the Yield Value measurement are explained in Technical Data Sheet Number 244 (Revision: 10/15/2007) from Lubrizol Advanced Materials, Inc. Low yield values (<50 dyns/cm$^2$) are indicative of smooth and Newtonian-like flow properties.

**Critical Micelle Concentration Protocol**

The CMC of an aqueous solution of test sample is determined by measuring the surface tension of the sample at ambient room temperature over a range of progressively increasing surfactant concentrations (Forward Titration Tensiometry Test). The test sample is sequentially dosed with a surfactant dosing solution using the Krüss K100 automatic tensiometer (Krüss USA, Matthews, N.C.) integrated with a 765 Dosimat automated dosing meter and personal computer loaded with LabDesk™ measurement and analysis software (data were collected using version 3.1 and analyzed using version 3.2 with the CMC add-on program). The test is conducted via the Wilhelmy plate method (Holmberg, K.; Jonsson, B.; Kronberg, B.; Lindman, B. Surfactants and Polymers in Aqueous Solution, Wiley & Sons, p. 347) using a platinum plate (19.9 mm wide x 10 mm high x 0.2 mm thick) and SV20 glass sample vessel (66.5 mm diameter x 35.0 mm high; volume = 121.563 ml).

A 100 g test sample solution is prepared by weighing an amount equal to 500 mg (polymer solids) of the non-crosslinked, linear acrylic copolymer mitigant of the disclosed technology into a suitable container. HPLC grade water (EMD Chemicals Inc, NJ) is added to the copolymer mitigant in an amount sufficient to bring the weight of the solution to 100 g. The test sample can be tested in the unneutralized state or can be neutralized to a desired pH value or degree of neutralization depending on the test parameters.

The surfactant dosing solution is prepared by dispersing a sufficient amount of the surfactant in HPLC grade water to obtain a stock concentration of 5750 mg/L of surfactant actives in HPLC grade water. The supply line of the dosimeter is placed into the dosing solution.

Fifty ml of the test sample is measured into the sample vessel equipped with a magnetic stir bar and is placed onto the tensiometer platform for surfactant dosing and surface tension analysis. Forty-two sequential surfactant doses of increasing concentration are metered into the test sample, increasing the surfactant concentration from 0 mg/L in the initial dose to approximately 3255 mg/L after the final dose. Subsequent to each metered dose, the surface tension of the test solution is measured by the tensiometer. Following each dosing cycle the solution is stirred for at least 3 minutes before the surface tension measurement is taken. From the data generated, a plot of measured surface tension versus concentration is created, giving a surface tension profile of the test sample at specific surfactant concentrations. The curve that is produced exhibits a sharp break at a particular point below which surface tension is not significantly affected by surfactant concentration. The surfactant concentration at this break point corresponds to the CMC. The approximate CMC point is located at the intersection of straight lines drawn through the data points obtained for concentration dependent portion of the plot and through the data points obtained for the concentration independent section of the plot.

**Molecular Weight Determination**

The number average ($M_n$) of the polymer samples are determined via the GPC method using a PL-220 high temperature GPC instrument manufactured by Polymer Laboratories. The instrument is integrated with a Compaq Dell OptiPlex GX270 computer with Waters Empower Pro LC/GPC software. Approximately 0.02 g polymer sample is dissolved in 5 ml of dimethyl actamide (DMAc), containing 250 ppm BHT and 0.05 molar $NaNO_3$. The test sample solution is gently shaken for about two hours and filtered with a 0.45 $\mu$m PTFE disposable disc filter. The chromatographic conditions are:

| | |
|---|---|
| Mobile phase: | DMAc, with 250 ppm BHT and 0.05m $NaNO_3$, 70°C, 1.0 ml/min. |
| Sample size: | 100$\mu$l |
| Column set: | PLgel (Guard + 2 x Mixed-B), all 10$\mu$m, in series |
| Detector: | Refractive Index Detector |
| Calibration standard: | PMMA |

**Emulsion Polymerization Method**

A general emulsion polymerization procedure for the preparation of the low molecular weight, non-crosslinked, linear acrylic copolymers of the present technology is provided as follows. A monomer emulsion is prepared in a first reactor equipped with a nitrogen inlet and a mixing agitator by combining the desired amount of each monomer with water that contains an emulsifying amount of an anionic surfactant. The components are mixed under a nitrogen atmosphere to until an emulsion is obtained. To a second reactor equipped with a mixing agitator, nitrogen inlet and feed pumps are added a desired amount of water and optional additional anionic surfactant. The contents are heated under a nitrogen atmosphere with mixing agitation. After the second reactor reaches a temperature in the range of about 70 to 95°C, a desired amount of a free radical initiator is injected into the solution in the second reactor. The monomer emulsion from the first reactor is then metered into the second reactor over a period ranging from about 1 to about 4 hours at a controlled reaction temperature in the range of about 80 to 90°C. After completion of the monomer addition, an additional quantity of free radical initiator can be added to the second reactor, if desired. The resulting reaction mixture is held at a temperature of about 85 to 95°C for a time period sufficient to complete the polymerization reaction, typically about 90 minutes. The resulting polymer emulsion can then be cooled and discharged from the reactor.

**Example 1 (comparative)**

Into an agitator equipped first reactor containing 112.0 grams of deionized water (D.I.) and 21.33 grams of sodium lauryl sulfate (30% active in water wt./wt.), 230.8 grams of ethyl acrylate (EA), 160 grams of methacrylic acid (MAA), 1.2 grams of n-dodecyl mercaptan (n-DDM) were added under nitrogen atmosphere and mixed at 900 rpm to form a monomer emulsion. To an agitator equipped second reactor were added 680 grams of deionized water and 4.0 grams of sodium lauryl sulfate (30% active in water wt./wt.). The contents of the second reactor were heated with agitation (300 rpm) under a nitrogen atmosphere. When the contents of the second reactor reached a temperature of 84°C, 8 grams of an ammonium persulfate solution (8.0% aqueous solution wt./wt.) was injected into the heated surfactant solution. The monomer emulsion from the first reactor was gradually metered into the second reactor at a feed rate of 3.1 g/min. over a period of 180 minutes at a reaction temperature maintained at 85°C. With the emulsion monomer feed, 1.4% ammonium persulfate solution (aqueous solution wt./wt.) was simultaneously metered into the reaction mixture in the second reactor. After completion of the monomer addition, an additional quantity of free radical initiator can be added to the second reactor, if desired, or the resulting reaction mixture can be held at a temperature of about 90°C for an additional two and half hours to ensure that residual monomer is polymerized. The resulting polymer emulsion product is cooled to room temperature (approximately 25°C), discharged from the reactor and recovered. The recovered emulsion polymer latex had a total polymer solids (active polymer) content of about 30 wt.%. The monomer components (wt.% based on the total monomer weight) are set forth in Table 1.

Examples 2 to 6

The monomer components set forth in Table 1 were polymerized as described in Example 1 except that the methacrylic acid levels for each example was changed (based on the desired theoretical acid number) as set forth in Table 1. The polymer solids (active polymer) content of the emulsion latexes were adjusted from about 31 wt.% to about 18 wt.% by increasing the levels of methacrylic acid.

Table 1

| Example | Total Solids (wt.%) | EA (wt.%) | MAA (wt.%) | n-DDM (wt.%)[2] | Theoretical Acid No. (m.eq.KOH/g) |
|---------|---------------------|-----------|------------|-----------------|-----------------------------------|
| 1[1]    | 31.4                | 60        | 40.0       | 0.3             | 261                               |
| 2[1]    | 28.8                | 50        | 50.0       | 0.3             | 326                               |
| 3       | 25.0                | 45        | 55.0       | 0.3             | 359                               |
| 4       | 22.1                | 40        | 60.0       | 0.3             | 391                               |
| 5       | 20.0                | 35        | 65.0       | 0.3             | 424                               |
| 6       | 17.9                | 30        | 70.0       | 0.3             | 457                               |
| [1]Comparative<br>[2]Parts by wt. based on 100 wt. parts of monomer | | | | | |

Example 7

The polymers of Examples 1 to 6 were added to aliquots of Johnson's® Baby Shampoo purchased at retail (lot number: 30002454; pH 6.72; viscosity 4,280 mPa.s; and NTU 3.29 and lot number 30002468; pH 6.53; viscosity 2,950 mPa.s; and NTU 2.56) to determine the effect the polymers had on the clarity properties of the shampoo under varying acidic pH conditions. The label on the shampoo product container listed the following ingredients (INCI nomenclature): Water, Cocamidopropyl Betaine, PEG 80 Sorbitan Laurate, Sodium Trideceth Sulfate, PEG 150 Distearate, Fragrance, Polyquaternium-10, Tetrasodium EDTA, Quaternium-15, Citric Acid, D&C Yellow 10, D&C Orange 4, Sodium Hydroxide.

Each of the emulsion polymers of Examples 1 to 6 at a concentration of 1.8 wt.% active polymer solids (based on the weight of the shampoo composition) was separately added with stirring to 600 gram samples of the baby shampoo. 100 g aliquot samples of each of the 600 g polymer modified shampoo samples were transferred into 6 dram vials. The pH of the contents of each vial was raised to 5.8 to 6.0 with an 18% sodium hydroxide solution to neutralize the polymer. The aliquots were then back-acid titrated with a 50% citric acid solution to a pH value of approximately 5.0 (Sample 1), approximately 4.5 (Sample 2) and approximately 4.0 (Sample 3). The samples were allowed to rest for 18 hours before measurements were taken. The results are reported in Table 2.

Table 2

| Polymer Example No. | 1[1] | 2[1] | 3 | 4 | 5 | 6 |
|---|---|---|---|---|---|---|
| Acid No. Theoretical (mEq. KOH/q) | 261 | 326 | 359 | 391 | 424 | 457 |
| Sample 1 | | | | | | |
| pH | 5.09 | 5.12 | 5.05 | 5.20 | 5.07 | 5.09 |
| Viscosity | 2,470 | 4,380 | 3,380 | 6,110 | 3,210 | 5,480 |
| Turbidity | 10.1 | 4.07 | 4.38 | 3.96 | 3.96 | 4.72 |
| Sample 2 | | | | | | |
| pH | 4.47 | 4.50 | 4.61 | 4.49 | 4.59 | 4.60 |
| Viscosity | 3,225 | 3,420 | 2,960 | 4,240 | 2,370 | 3,890 |
| Turbidity | 19.1 | 4.90 | 4.64 | 4.10 | 4.10 | 4.63 |
| Sample 3 | | | | | | |
| pH | 4.05 | 4.00 | 4.11 | 4.07 | 4.07 | 4.10 |
| Viscosity | 3,165 | 3,160 | 2,690 | 3,590 | 2,010 | 3,140 |
| Turbidity | 25.2 | 5.49 | 4.85 | 4.26 | 4.08 | 5.09 |
| [1]Comparative | | | | | | |

Example 8

Samples 1 and 2 of the baby shampoo dosed with the polymer of comparative Example 1 which were prepared in Example 7 were allowed to age at room temperature to evaluate turbidity over an extended period. Sample 0 (not reported in Example 7) was prepared concurrently with Samples 1 and 2 (polymer of Example 1) and back-acid titrated to a pH value of approximately 5 according to the method described in Example 7. The neat shampoo (containing no polymer) was evaluated as a benchmark comparison and showed only a minor change in turbidity. Table 3 reports the turbidity values of samples modified with the polymer of Example 1 at pH values of approximately 5.5 (Sample 0), 5.0 (Sample 1) and 4.5 (Sample 2) and aged at ambient room temperature (20-25°C). Table 4 summarizes the turbidity values for the neat baby shampoo lot numbers aged at ambient room temperature (20-25°).

Table 3

| Polymer Example No. 1[1] | Sample 0[2] | Sample 1[3] | Sample 2[3] |
|---|---|---|---|
| pH | 5.5 | 5.0 | 4.5 |
| Initial NTU[4] | 5.57 | 10.1 | 19.1 |
| NTU (aged 34 days at ambient room temperature) | 10.9 | 39.7 | 76.6 |
| NTU aged 49 Days at ambient room temperature) | 11.8 | 44.9 | 89 |
| [1]Comparative<br>[2]Prepared concurrently with Samples 1 and 2 of Example 7 (Polymer No. 1)<br>[3]Samples 1 and 2 reported in Table 2 of Example 7 (Polymer No. 1)<br>[4]Measured 18 hours after back-acid titration | | | |

Table 4

| Johnson's Baby Shampoo (neat) | Lot Number 30002454 | Lot Number 30002468 |
|---|---|---|
| NTU (initial) | 3.29 | 2.56 |
| NTU (@ 27 days at ambient RT) | 3.89 | 2.57 |
| NTU (@ 43 days at ambient RT) | 3.84 | 2.61 |

(continued)

| Johnson's Baby Shampoo (neat) | Lot Number 30002454 | Lot Number 30002468 |
|---|---|---|
| NTU (@ 12 weeks at ambient RT) | 4.17 | 2.50 |

After approximately 30 days of ambient room temperature (20-25°C) aging it was observed that the turbidity of baby shampoo samples modified with the polymer of Example 1 prepared from 50 wt.% MAA (acid no. $\leq$326 mEq KOH/g) increased significantly for compositions having pH values (pH $\leq$5).

Example 9

A shampoo composition containing the polymer of comparative Example 2 (Sample 2) was prepared as in Example 7 (back-acid titrated to approximately pH 4.5). The sample was aged in a temperature controlled oven at 45°C for a period of 16 weeks. An initial turbidity measurement was taken at the onset of the evaluation and at 4 weeks and 16 weeks. The initial turbidity measurement was taken immediately after the sample reached 45°C, removed from the oven and allowed to cool to ambient room temperature. The 4 and 16 week measurements were taken immediately when the sample was removed from the oven and again after the sample cooled to ambient room temperature. The results are reported in Table 5.

Table 5

| Polymer Example No. 2[1] | Aging Condition (45°C) | NTU Measured at 45°C (immediately after removing sample from oven) | NTU Measured at RT (oven aged sample after reaching RT) |
|---|---|---|---|
| Sample 2 (pH 4.5) | Initial | -- | 4.9 |
| | 4 weeks | 31.3 | 17.1 |
| | 16 weeks | 126.0 | 42.2 |
| [1]Comparative | | | |

While the polymer of Comparative Example 2 (containing 50 wt.% MAA; acid no. $\approx$ 326 mEq) provides acceptable turbidity in the baby shampoo composition at low pH values as demonstrated in Table 2 of Example 7, it is evident that the turbidity properties are not stable when the composition is exposed to elevated temperatures over an extended period. Upon aging at 45°C, samples became hazy with increased NTU values over time.

Example 10

Baby shampoo samples were prepared from the polymer of the present technology as set forth in Example 7. The polymer dosed shampoo compositions were back-acid titrated to a pH of approximately 4.5. Each of the samples was then oven aged at 45°C as set forth in Example 9 for a period of 11 weeks. An initial turbidity measurement was taken at the onset of the evaluation and at 3.5 weeks and 11 weeks. The initial turbidity measurement was taken immediately after the sample reached 45°C, removed from the oven and allowed to cool to ambient room temperature. The 3.5 and 11 week measurements were taken immediately when the sample was removed from the oven and again after the sample cooled to ambient room temperature. The results are reported in Table 6.

Table 6

| Polymer Example No. | Aging Condition (45°C) | NTU Measured at 45°C (immediately after removing sample from oven) | NTU Measured at RT (oven aged sample after reaching RT) |
|---|---|---|---|
| Example 3 (pH 4.5) | Initial | 4.93 | 4.93 |
| | 3.5 weeks | 15.4 | 10.1 |
| | 11 weeks | 11.1 | 10.9 |
| | | | |

(continued)

| Polymer Example No. | Aging Condition (45°C) | NTU Measured at 45°C (immediately after removing sample from oven) | NTU Measured at RT (oven aged sample after reaching RT) |
|---|---|---|---|
| Example 4 (pH 4.5) | Initial | 4.03 | 4.03 |
| | 3.5 weeks | 6.59 | 5.39 |
| | 11 weeks | 8.84 | 7.29 |
| | | | |
| Example 5 (pH 4.5) | Initial | 4.08 | 4.08 |
| | 3.5 weeks | 4.66 | 4.30 |
| | 14 weeks | 8.31 | 7.6 |
| | | | |
| Example 6 (pH 4.5) | Initial | 4.65 | 4.65 |
| | 3.5 Weeks at 45C | 5.05 | 4.48 |
| | 14 weeks at 45C | 9.66 | 9.04 |

Surprisingly, the baby shampoo formulations containing the irritation mitigant polymers of the present technology maintained very high clarity with desirable low NTU values (< 15) at low pH values and even after aging at elevated temperature (45°C) for a prolonged period of 11 to 14 weeks. Accelerated oven aging at 45°C for 12 weeks corresponds to a 1 year stable shelf life for a product.

Example 11 (comparative)

Polymer EX-968 (manufactured and supplied by the Noveon Consumer Specialties Division of Lubrizol Advanced Materials, Inc.) a low molecular weight, non-crosslinked, linear emulsion polymer (30.9 wt.% active solids) having a methacrylic acid:ethyl acrylate ratio of 25:75%, based on the total weight of all of the monomers in the polymerization medium and having an $M_n$ of from about 15,000 to about 40,000 Daltons was dosed into Johnson's® Baby Shampoo to determine the effect of reduced pH on the turbidity of the dosed sample. The results are reported in Table 7.

Table 7

| Commercial Baby Shampoo | Neat | Dosed |
|---|---|---|
| EX-968 Polymer Emulsion (g) (30.9 wt.% active polymer solids) | -- | 6.99 (2.16 g active polymer solids) |
| Shampoo wt. (g) | -- | 113.01 |
| NaOH (18%) | -- | 0.21 |
| pH | 6.37 (as is) | 5.86 (final) |
| Viscosity (mPa·s) | 3,430 | 1,620 |
| Viscosity (mPa·s) @ 0.5 rpm | 4,400 | 2,400 |
| Viscosity (mPa·s) @ 1.0 rpm | 3,800 | 2,000 |
| Yield Value | 6 | 4 |
| Turbidity (NTU) | 3.66 | Indeterminable[1] |
| Appearance | Clear (amber yellow) | Opaque (dark yellow) |
| [1]Sample was above the upper measurement limit of the turbidity measuring instrument - too opaque to test | | |

Johnson's® Baby Shampoo having the identical ingredients described in Example 7 was added to a 150 ml beaker and stirred. The EX-968 polymer emulsion was dosed into the shampoo at an active polymer solids level of 1.8 wt.%, based on the weight of the total composition. The initial pH of the neat shampoo (before dosing) was 6.37. After dosing the neat shampoo with the emulsion polymer the pH of the dosed shampoo dropped below about 5.8. The pH of dosed shampoo composition was subsequently adjusted with 18% NaOH to a final value of 5.86. The sample was allowed to rest for 18 hours, centrifuged to remove air bubbles, and evaluated for turbidity. As is apparent from the results in Table 7, reducing the pH of the shampoo composition dosed with polymer EX-968 deleteriously effects clarity.

Example 12

The CMC of a surfactant composition containing the anionic surfactant, sodium trideceth sulfate (TDES) and the non-crosslinked, linear acrylic copolymers of the present technology, is determined by plotting tensiometry data generated by the Kruss K100 automatic tensiometer. The CMC methodology as described in the CMC protocol was utilized. The titrations are conducted for each polymer at 500 mg/L without neutralization. The CMC values for each polymer at respective acid numbers are set forth in Table 8 along with the control (surfactant TDES with no polymer). The TDES surfactant used in these experiments exhibited CMC values ranging 190-198 mg/L, which are slightly higher than the reported value of 136 mg/L in U.S. Patent No. 7,803,403. All non-crosslinked, linear acrylic copolymers in Table 8 exhibit increasing CMC values when titrated with TDES, indicating a strong polymer association with TDES surfactant.

Table 8

| Polymer Example No. | Acid No. | CMC (mg/L) |
|---|---|---|
| TDES (Control Surfactant) | -- | 190-198 |
| 1[1] | 261 | 749.5 |
| 2[1] | 326 | 790.3 |
| 3 | 359 | 802.3 |
| 4 | 391 | 885.4 |
| 5 | 424 | 919.6 |
| 6 | 457 | 1007.2 |
| [1]Comparative polymer | | |

While certain representative embodiments and details have been shown for the purpose of illustrating the subject technology, it will be apparent to those skilled in this art that various changes and modifications can be made therein without departing from the scope of the subject disclosed technology. In this regard, the scope of the disclosed technology is to be limited only by the following claims.

**Claims**

1. An optically stable aqueous surfactant composition comprising:

   a) from 0.05 to 6 wt%, on active polymer basis, of a low molecular weight, non-crosslinked, linear emulsion polymer having a number average molecular wt. ranging from 10,000 to 100,000 Daltons, measured as indicated in the Methods Description section of the specification, said polymer comprising from 51 to 75 wt.% of polymerized residues of at least one ethylenically unsaturated $C_3$-$C_6$ carboxylic acid monomer, from 25 to 49 wt.% of polymerized residues of a at least one $C_1$-$C_4$ alkyl (meth)acrylate monomer, and from 0 to 1 wt.% of a residue of a chain transfer agent, based on 100 wt. parts of monomer; and
   b) at least one surfactant.

2. A composition of claim 1 wherein said at least one carboxylic acid monomer is selected from acrylic acid, methacrylic acid, itaconic acid, citraconic acid, maleic acid, maleic anhydride, fumaric acid, crotonic acid, aconitic acid, and mixtures thereof.

3. A composition of any one of the previous claims wherein said at least one $C_1$-$C_4$ alkyl (meth)acrylate monomer is selected from methyl (meth)acrylate, ethyl (meth)acrylate, n-butyl (meth)acrylate, t-butyl (meth)acrylate, and mixtures thereof.

4. A composition of any one of the previous claims wherein said alkyl mercaptan chain transfer agent is a selected from a $C_1$-$C_{18}$ alkyl mercaptan.

5. A composition of any one of the previous claims wherein said chain transfer agent is selected from octyl mercaptan, n-dodecyl mercaptan, t-dodecyl mercaptan, hexadecyl mercaptan, octadecyl mercaptan.

6. A composition of any one of the previous claims wherein said emulsion polymer comprises 55 to 70 wt.% of a residue polymerized from methacrylic acid and 45 to 30 wt.% of a residue polymerized from a monomer selected from ethyl acrylate, n-butyl acrylate, and mixtures thereof.

7. A composition of any one of the previous claims wherein said emulsion polymer comprises a residue polymerized from methacrylic acid, a residue polymerized from ethyl acrylate, and a residue of a chain transfer agent selected from a $C_1$-$C_{18}$ alkyl mercaptan.

8. A composition of any of the previous claims wherein said surfactant is selected from an anionic surfactant, a nonionic surfactant, an amphoteric surfactant, and mixtures thereof, preferably wherein said surfactant is present in an amount ranging from 1 to 30 wt.% based on the total wt. of the composition.

9. A composition of any one of the previous claims wherein said composition further comprises a food grade acid preservative, food grade acid preservative salts, and mixtures thereof, preferably wherein said food grade acid preservative is selected from benzoic acid, acetic acid, propionic acid, sorbic acid, salicylic acid, lactic acid, citric acid, fumaric acid, malic acid, the calcium, potassium and sodium salts thereof, sodium benzoate, potassium sorbate, sodium propionate, calcium dipropionate, and mixtures thereof.

10. A method for stabilizing the optical clarity properties of a low pH aqueous surfactant containing composition, said method comprising adding from 0.05 to 6 wt%, on active polymer basis, of a low molecular weight, non-crosslinked, linear emulsion acrylic polymer having a number average molecular wt. ranging from 10,000 to 100,000 Daltons to said surfactant composition, wherein said emulsion polymer comprises from 51 to 75 wt.% of polymerized residues of at least one ethylenically unsaturated $C_3$-$C_6$ carboxylic acid monomer, from 25 to 49 wt.% of polymerized residues of a at least one $C_1$-$C_4$ alkyl (meth)acrylate monomer, and from 0 to 0.5 wt.% of a residue of a chain transfer agent, based on 100 wt. parts of monomer, wherein the molecular weight has been measured as indicated in the section Methods Description of the specification.

11. A method of claim 10 wherein said emulsion polymer has a molecular weight of from 25,000 to 80,000 Daltons ($M_n$), from 40,000 and 75,000 Daltons ($M_n$), and from 50,000 to 70,000 Daltons ($M_n$) and/or wherein the pH of said composition ranges from 2 to 5.5, from 2.5 to 5, from 3 to 4.5, from 3.5 to 4.

12. A method for preparing a mild, optically stable surfactant composition comprising:

   a) combining from 0.05 to 6 wt%, on active polymer basis, of a low molecular weight, non-crosslinked, linear emulsion acrylic polymer, having a number average molecular weight of 10,000 to 100,000 Daltons, with a surfactant composition, wherein said emulsion polymer comprises from 51 to 75 wt.% of polymerized residues of at least one ethylenically unsaturated $C_3$-$C_6$ carboxylic acid monomer, from 25 to 49 wt.% of polymerized residues of a at least one $C_1$-$C_4$ alkyl (meth)acrylate monomer, and from 0 to 0.5 wt.% of a residue of a chain transfer agent, based on 100 wt. parts of monomer;
   b) optionally neutralizing said surfactant composition with an alkaline neutralizing agent to a pH ranging from 6 to 8;
   c) back-acid titrating said surfactant composition with an acidifying agent to a pH ranging from 2 to 5.5; wherein the molecular weight has been measured as indicated in the Methods Description section of the specification.

13. A method of claim 12 wherein said acidifying agent is selected from a food grade acid preservative, salts thereof, and mixtures thereof, preferably wherein said food grade acid preservative is selected from is selected from benzoic acid, acetic acid, propionic acid, sorbic acid, salicylic acid, lactic acid, citric acid, fumaric acid, malic acid, the calcium, potassium and sodium salts thereof, sodium benzoate, potassium sorbate, sodium propionate, calcium dipropionate, and mixtures thereof.

**14.** A method of any one of claims 10 to 13 wherein the surfactant composition comprises an anionic surfactant, an amphoteric surfactant, and mixtures thereof.

**Patentansprüche**

**1.** Optisch stabile, wässrige Tensidzusammensetzung, umfassend:

a) 0,05 bis 6 Gew.-%, auf Basis des aktiven Polymers, eines niedermolekularen, nicht vernetzten, linearen Emulsionspolymers mit einem Zahlenmittel der Molekularmasse von 10.000 bis 100.000 Dalton, gemessen nach den Angaben im Abschnitt der Verfahrensbeschreibung der Patentschrift, wobei das Polymer 51 bis 75 Gew.-% polymerisierte Reste mindestens eines ethylenisch ungesättigten $C_3$-$C_6$-Carbonsäure-Monomers, 25 bis 49 Gew.-% polymerisierte Reste mindestens eines $C_1$-$C_4$-Alkyl(meth)acrylat-Monomers und 0 bis 1 Gew.-% eines Rests eines Kettenübertragungsmittels, basierend auf 100 Gewichtsanteilen des Monomers, umfasst; und
b) mindestens ein Tensid.

**2.** Zusammensetzung nach Anspruch 1, wobei das mindestens eine Carbonsäure-Monomer aus Acrylsäure, Methacrylsäure, Itaconsäure, Citraconsäure, Maleinsäure, Maleinsäureanhydrid, Fumarsäure, Crotonsäure, Aconitsäure und Mischungen davon ausgewählt ist.

**3.** Zusammensetzung nach einem der vorhergehenden Ansprüche, wobei das mindestens eine $C_1$-$C_4$-Alkyl(meth)acrylat-Monomer aus Methyl(meth)acrylat, Ethyl(meth)acrylat, n-Butyl(meth)acrylat, t-Butyl(meth)acrylat und Mischungen davon ausgewählt ist.

**4.** Zusammensetzung nach einem der vorhergehenden Ansprüche, wobei das Alkylmercaptan-Kettenübertragungsmittel aus einem $C_1$-$C_{18}$-Alkylmercaptan ausgewählt ist.

**5.** Zusammensetzung nach einem der vorhergehenden Ansprüche, wobei das Kettenübertragungsmittel aus Octylmercaptan, n-Dodecylmercaptan, t-Dodecylmercaptan, Hexadecylmercaptan, Octadecylmercaptan ausgewählt ist.

**6.** Zusammensetzung nach einem der vorhergehenden Ansprüche, wobei das Emulsionspolymer 55 bis 70 Gew.-% eines aus Methacrylsäure polymerisierten Rests und 45 bis 30 Gew.-% eines Rests, polymerisiert aus einem Monomer, das aus Ethylacrylat, n-Butylacrylat und Mischungen davon ausgewählt ist, umfasst.

**7.** Zusammensetzung nach einem der vorhergehenden Ansprüche, wobei das Emulsionspolymer einen Rest, polymerisiert aus Methacrylsäure, einen Rest, polymerisiert aus Ethylacrylat, und einen Rest eines Kettenübertragungsmittels, ausgewählt aus einem $C_1$-$C_{18}$-Alkylmercaptan, umfasst.

**8.** Zusammensetzung nach einem der vorhergehenden Ansprüche, wobei das Tensid aus einem anionischen Tensid, einem nichtionischen Tensid, einem amphoteren Tensid und Mischungen davon ausgewählt ist, wobei das Tensid basierend auf dem Gesamtgewicht der Zusammensetzung bevorzugt in einer Menge von 1 bis 30 Gew.-% vorliegt.

**9.** Zusammensetzung nach einem der vorhergehenden Ansprüche, wobei die Zusammensetzung ferner ein Säure-Konservierungsmittel in Lebensmittelqualität, Säuresalz-Konservierungsmittel in Lebensmittelqualität und Mischungen davon umfasst, wobei das Säure-Konservierungsmitteln in Lebensmittelqualität bevorzugt aus Benzoesäure, Essigsäure, Propionsäure, Sorbinsäure, Salicylsäure, Milchsäure, Citronensäure, Fumarsäure, Äpfelsäure, den Calcium-, Kalium- und Natriumsalzen davon, Natriumbenzoat, Kaliumsorbat, Natriumpropionat, Calciumdipropionat und Mischungen davon ausgewählt ist.

**10.** Verfahren zum Stabilisieren der Eigenschaften der optischen Klarheit einer wässrigen, tensidhaltigen Zusammensetzung mit niedrigem pH-Wert, wobei das Verfahren ein Zugeben von 0,05 bis 6 Gew.-% , auf Basis des aktiven Polymers, eines niedermolekularen, nicht vernetzten, linearen Emulsionsacrylpolymers mit einem Zahlenmittel der Molekularmasse von 10.000 bis 100.000 Dalton zu der Tensidzusammensetzung umfasst, wobei das Emulsionspolymer 51 bis 75 Gew-.% polymerisierte Reste mindestens eines ethylenisch ungesättigten $C_3$-$C_6$-Carbonsäure-Monomers, 25 bis 49 Gew.-% polymerisierte Reste mindestens eines $C_1$-$C_4$-Alkyl(meth)acrylat-Monomers und 0 bis 0,5 Gew.-% eines Rests eines Kettenübertragungsmittels basierend auf 100 Gewichtsteilen Monomer umfasst, wobei die Molekularmasse nach den Angaben im Abschnitt der Verfahrensbeschreibung der Patentschrift gemessen wurde.

**11.** Verfahren nach Anspruch 10, wobei das Emulsionspolymer eine Molekularmasse von 25.000 bis 80.000 Dalton ($M_n$), von 40.000 bis 75.000 Dalton ($M_n$) und von 50.000 bis 70.000 Dalton ($M_n$) hat und/oder wobei der pH-Wert der Zusammensetzung im Bereich von 2 bis 5,5, von 2,5 bis 5, von 3 bis 4,5 oder von 3,5 bis 4 liegt.

**12.** Verfahren zum Herstellen einer milden, optisch stabilen Tensid-Zusammensetzung, umfassend:

a) Kombinieren von 0,05 bis 6 Gew.-% , auf Basis des aktiven Polymers, eines niedermolekularen, nicht vernetzten, linearen Emulsionsacrylpolymers auf mit einem Zahlenmittel der Molekularmasse von 10.000 bis 100.000 Dalton mit einer Tensid-Zusammensetzung, wobei das Emulsionspolymer 51 bis 75 Gew.-% polymerisierte Reste mindestens eines ethylenisch ungesättigten $C_3$-$C_6$-Carbonsäure-Monomers, 25 bis 49 Gew.-% polymerisierte Reste mindestens eines $C_1$-$C_4$-Alkyl(meth)acrylat-Monomers und 0 bis 0,5 Gew.-% eines Rests eines Kettenübertragungsmittels basierend auf 100 Gewichtsanteilen des Monomers umfasst;
b) wahlweises Neutralisieren der Tensid-Zusammensetzung mit einem alkalischen Neutralisationsmittel auf einen pH-Wert im Bereich von 6 bis 8;
c) saures Rücktitrieren der Tensid-Zusammensetzung mit einem ansäuernden Mittel auf einen pH-Wert im Bereich von 2 bis 5,5; wobei die Molekularmasse nach der im Abschnitt der Verfahrensbeschreibung der Patentschrift gemessen wurde.

**13.** Verfahren nach Anspruch 12, wobei das ansäuernde Mittel aus einem Säure-Konservierungsmittel in Lebensmittelqualität, Salzen davon und Mischungen davon ausgewählt ist, wobei das Säure-Konservierungsmittel in Lebensmittelqualität bevorzugt aus Benzoesäure, Essigsäure, Propionsäure, Sorbinsäure, Salicylsäure, Milchsäure, Citronensäure, Fumarsäure, Äpfelsäure, Calcium-, Kalium- und Natriumsalzen davon, Natriumbenzoat, Kaliumsorbat, Natriumpropionat, Calciumdipropionat und Mischungen davon ausgewählt ist.

**14.** Verfahren nach einem der Ansprüche 10 bis 13, wobei die Tensid-Zusammensetzung ein anionisches Tensid, ein amphoteres Tensid und Mischungen davon umfasst.

**Revendications**

**1.** Composition tensioactive aqueuse optiquement stable, comprenant :

a) 0,05 à 6 % en poids, sur la base d'un polymère actif, d'un polymère en émulsion linéaire non réticulé de faible poids moléculaire ayant un poids moléculaire moyen en nombre compris entre 10 000 et 100 000 daltons, mesuré comme indiqué dans la section Description des méthodes de la description, ledit polymère comprenant 51 à 75 % en poids de résidus polymérisés d'au moins un monomère d'acide carboxylique en $C_3$-$C_6$ à insaturation éthylénique, 25 à 49 % en poids de résidus polymérisés d'au moins un monomère de (méth)acrylate d'alkyle en $C_1$-$C_4$ et 0 à 1 % en poids d'un résidu d'un agent de transfert de chaîne, sur la base de 100 % en poids de parties de monomère ; et
b) au moins un tensioactif.

**2.** Composition selon la revendication 1, dans laquelle ledit au moins un monomère d'acide carboxylique est choisi parmi l'acide acrylique, l'acide méthacrylique, l'acide itaconique, l'acide citraconique, l'acide maléique, l'anhydride maléique, l'acide fumarique, l'acide crotonique, l'acide aconitique et leurs mélanges.

**3.** Composition selon l'une quelconque des revendications précédentes, dans laquelle ledit au moins un monomère de (méth)acrylate d'alkyle en $C_1$-$C_4$ est choisi parmi le (méth)acrylate de méthyle, le (méth)acrylate d'éthyle, le (méth)acrylate de n-butyle, le (méth)acrylate de t-butyle et leurs mélanges.

**4.** Composition selon l'une quelconque des revendications précédentes, dans laquelle ledit agent de transfert de chaîne d'alkylmercaptan est choisi à partir d'un alkylmercaptan en $C_1$-$C_{18}$.

**5.** Composition selon l'une quelconque des revendications précédentes, dans laquelle ledit agent de transfert de chaîne est choisi parmi l'octylmercaptan, le n-dodécylmercaptan, le t-dodécylmercaptan, l'hexadécylmercaptan, l'octadécylmercaptan.

**6.** Composition selon l'une quelconque des revendications précédentes, dans laquelle ledit polymère en émulsion comprend 55 à 70 % en poids d'un résidu polymérisé à partir d'acide méthacrylique et 45 à 30 % en poids d'un résidu polymérisé à partir d'un monomère choisi parmi l'acrylate d'éthyle, l'acrylate de n-butyle et leurs mélanges.

**7.** Composition selon l'une quelconque des revendications précédentes, dans laquelle ledit polymère en émulsion comprend un résidu polymérisé à partir d'acide méthacrylique, un résidu polymérisé à partir d'acrylate d'éthyle et un résidu d'un agent de transfert de chaîne choisi à partir d'un alkylmercaptan en $C_1$-$C_{18}$.

**8.** Composition selon l'une quelconque des revendications précédentes, dans laquelle ledit tensioactif est choisi parmi un tensioactif anionique, un tensioactif non ionique, un tensioactif amphotère et leurs mélanges, ledit tensioactif étant de préférence présent en une quantité comprise entre 1 et 30 % en poids sur la base du poids total de la composition.

**9.** Composition selon l'une quelconque des revendications précédentes, dans laquelle ladite composition comprend en outre un conservateur d'acide alimentaire, des sels de conservateur d'acide alimentaire et leurs mélanges, ledit conservateur d'acide alimentaire étant de préférence choisi parmi l'acide benzoïque, l'acide acétique, l'acide propionique, l'acide sorbique, l'acide salicylique, l'acide lactique, l'acide citrique, l'acide fumarique, l'acide malique, leurs sels de calcium, de potassium et de sodium, le benzoate de sodium, le sorbate de potassium, le propionate de sodium, le dipropionate de calcium et leurs mélanges.

**10.** Procédé destiné à la stabilisation des propriétés de transparence optique d'une composition contenant un tensioactif aqueux à faible pH, ledit procédé consistant à ajouter 0,05 à 6 % en poids, sur la base d'un polymère actif, d'un polymère acrylique en émulsion linéaire non réticulé de faible poids moléculaire ayant un poids moléculaire moyen en nombre compris entre 10 000 à 100 000 daltons à ladite composition tensioactive, ledit polymère en émulsion comprenant 51 à 75 % en poids de résidus polymérisés d'au moins un monomère d'acide carboxylique en $C_3$-$C_6$ à insaturation éthylénique, 25 à 49 % en poids de résidus polymérisés d'au moins un monomère de (méth)acrylate d'alkyle en $C_1$-$C_4$ et 0 à 0,5 % en poids d'un résidu d'un agent de transfert de chaîne, sur la base de 100 % en poids de parties de monomère, le poids moléculaire ayant été mesuré comme indiqué dans la section Description des méthodes de la description.

**11.** Procédé selon la revendication 10, dans lequel ledit polymère en émulsion a un poids moléculaire compris entre 25 000 et 80 000 daltons ($M_n$), entre 40 000 et 75 000 daltons ($M_n$) et entre 50 000 et 70 000 daltons ($M_n$) et/ou dans lequel le pH de ladite composition est compris entre 2 et 5,5, entre 2,5 et 5, entre 3 et 4,5, entre 3,5 et 4.

**12.** Procédé destiné à la préparation d'une composition tensioactive douce et optiquement stable, consistant à :

a) combiner 0,05 à 6 % en poids, sur la base d'un polymère actif, d'un polymère acrylique en émulsion linéaire non réticulé de faible poids moléculaire, ayant un poids moléculaire moyen en nombre compris entre 10 000 et 100 000 daltons, avec une composition tensioactive, ledit polymère en émulsion comprenant 51 à 75 % en poids de résidus polymérisés d'au moins un monomère d'acide carboxylique à insaturation éthylénique en $C_3$-$C_6$, 25 à 49 % en poids de résidus polymérisés d'au moins un monomère de (méth)acrylate d'alkyle en $C_1$-$C_4$ et 0 à 0,5 % en poids d'un résidu d'un agent de transfert de chaîne, sur la base de 100 % en poids de parties de monomère ;
b) neutraliser éventuellement ladite composition tensioactive avec un agent neutralisant alcalin à un pH compris entre 6 et 8 ;
c) réaliser un titrage en retour de l'acide avec ladite composition tensioactive au moyen d'un agent acidifiant à un pH compris entre 2 et 5,5 ; le poids moléculaire ayant été mesuré comme indiqué dans la section Description des méthodes de la description.

**13.** Procédé selon la revendication 12, dans lequel ledit agent acidifiant est choisi parmi un conservateur d'acide alimentaire, ses sels et leurs mélanges, ledit conservateur d'acide alimentaire étant de préférence choisi parmi l'acide benzoïque, l'acide acétique, l'acide propionique, l'acide sorbique, l'acide salicylique, l'acide lactique, l'acide citrique, l'acide fumarique, l'acide malique, leurs sels de calcium, de potassium et de sodium, le benzoate de sodium, le sorbate de potassium, le propionate de sodium, le dipropionate de calcium et leurs mélanges.

**14.** Procédé selon l'une quelconque des revendications 10 à 13, dans lequel la composition tensioactive comprend un tensioactif anionique, un tensioactif amphotère et leurs mélanges.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4132678 A
- US 4726915 A
- US 7803403 B
- US 8025902 B
- US 8293845 B
- EP 2402000 A
- US 6051541 A
- WO 9921530 A
- US 3929678 A
- US 4565647 A
- US 5456849 A
- US 5720964 A
- US 5858948 A
- US 7115550 B
- WO 2005023870 A
- US 7084104 B
- US 7098180 B

**Non-patent literature cited in the description**

- **SCHOENBERG.** Household & Personal Products Industry. *Baby Shampoo,* September 1979, vol. 60
- **WEBER, K.** New alternatives to paraben-based preservative blends. *Cosmetics & Toiletries,* 2005, vol. 120 (1), 57-62
- **WIECHERS, J.W.** Formulating at pH 4-5: How lower pH benefits the skin and formulations. *Cosmetics & Toiletries,* 2008, vol. 123 (12), 61-70
- **SCHWARTZ ; PERRY ; BERCH.** McCutcheon's Emulsifiers and Detergents. North American and International Editions
- *Invittox Protocol,* May 1994, vol. 86
- **MITCHELL C. SCHLOSSMAN.** The Chemistry and Manufacture of Cosmetics. Allured Publishing Corporation, 2000, vol. I, II
- **TODD ; BYERS.** Volatile Silicone Fluids for Cosmetics. *Cosmetics and Toiletries,* January 1976, vol. 91, 27-32
- **HOLMBERG, K. ; JONSSON, B. ; KRONBERG, B. ; LINDMAN, B.** Surfactants and Polymers in Aqueous Solution. Wiley & Sons, 347